# EUROPÄISCHE PATENTSCHRIFT

(11) **EP 3 154 469 B1**
(45) Veröffentlichungstag und Bekanntmachung des Hinweises auf die Patenterteilung: **14.11.2018**
(21) Anmeldenummer: 15730381.9
(22) Anmeldetag: 08.06.2015
(51) Int. Cl.: A61B 1/24, A61C 9/00

(54) **MESSVORRICHTUNG UND VERFAHREN ZUM DREIDIMENSIONALEN AUSMESSEN EINES MUNDRAUMS**
MEASURING APPARATUS AND METHOD FOR THREE-DIMENSIONAL MEASUREMENT OF AN ORAL CAVITY
DISPOSITIF DE MESURE ET PROCÉDÉ POUR EFFECTUER DES MESURES EN TROIS DIMENSIONS D'UNE CAVITÉ BUCCALE

(30) Priorität: 11.06.2014 CH 884142014
(43) Veröffentlichungstag der Anmeldung: 19.04.2017
(73) Patentinhaber: Quarz Partners AG, 8053 Zürich (CH)
(72) Erfinder: MÖRMANN, Werner, CH-8053 Zürich (CH); MEHL, Albert, 6340 Baar (CH)
(74) Vertreter: Sutter, Kurt
(86) Internationale Anmeldenummer: PCT/CH2015/000086
(87) Internationale Veröffentlichungsnummer: WO 2015/188286

(56) Entgegenhaltungen:
- JP-A- 2009 204 991
- US-A- 4 611 288
- US-A- 5 976 076

## Beschreibung

### Gebiet der Erfindung

Die Erfindung betrifft eine Messvorrichtung zum Ausmessen mindestens eines Teils eines Mundraums, insbesondere mindestens eines Teils einer Zahnreihe, sowie ein entsprechendes Verfahren.

### Hintergrund

Eine Übersicht über aktuelle intraorale 3D-Kamerasysteme liegt in Int J Comput Dent 2013; 16: 143-162 vor. Die dort beschriebenen Kameras verwenden eine Endoskop-artige Bauweise mit einem Kamerakörper und einem Zugangsschaft in den Mund, welcher an seinem Ende über ein optisches Fenster oder über Projektoren und Sensoren verfügt. Für serielle Aufnahmen werden diese Kameras freihändig und schwebend oder abgestützt über die Zahnreihe geführt. Erfahrungsgemäß eignen sich diese bekannten Scanner für die Vermessung von Teilzahnreihen.

Anwendungsprobleme bestehen bei Scannern dieser Bauart in der Erfassung von schwer einsehbaren Oberflächenanteilen beispielsweise von interdental nach mesial oder distal orientierten natürlichen Zahnflächen sowie von feinteiligen, teilweise vertikalen geometrischen Präparationsflächen. Dies auch bei Messungen von Flächen, die sich in den Zahnzwischenräumen unterhalb des Zahnäquators befinden, weil die mögliche Kiefer- und Mundöffnung das Ausrichten der Kamerablickrichtung mit dem starren Endoskopschaft besonders im Bereich der Molaren begrenzt. Dadurch ist die lückenlose Vermessung der schwer einsehbaren Oberflächen der Zahnreihen durch das manuelle Einstellen der erforderlichen Blickwinkel bei der bekannten Bauweise dieser Kameras erschwert.

Besonders schwierig ist die Erfassung interdentaler bukkaler und lingualer Seitenflächen, da sie freihändige Roll- und Kippbewegungen der Kamera bei gleichzeitiger, schwebender Bewegung entlang der Zahnreihen verlangt. Zudem muss die Kamera innerhalb des Schärfentiefenbereiches bewegt werden. Gleichzeitig sind die Wangen- und Zungenweichteile vom Messbereich abzuhalten und der Speichelfluss muss kontrolliert werden.

Speziell anforderungsreich erweist sich aus den genannten Gründen bei den bekannten Systemen die vollständige Vermessung der Zahnreihen des gesamten Gebisses mit den voll- oder teilbezahnten Ober- und Unterkiefern und die Vermessung des Gaumens sowie zahnloser Kiefer. Die vollständige Vermessung des Gebisses ist für die Fertigung prothetischer Rekonstruktionen und Prothesen sowie in der Kieferorthopädie für die Planung und Kontrolle der Therapie wichtig. Der Anwender soll deshalb in die Lage versetzt werden, die Messungen möglichst einfach und schnell zu erledigen. Angestrebt wird dabei, dass das Messystem der Kamera beim Scannen der Zahnreihen an die unterschiedliche anatomische Topologie der Kiefer bei voller, teilweiser oder fehlender Bezahnung anpassbar gestaltet ist, und dass es die Vermessung aus unterschiedlichen Raumwinkeln automatisch selbst durchführt und damit den Benutzer davon befreit, die schwierigen Einstellungen der für eine lückenlose Vermessung erforderlichen Raumwinkel manuell vorzunehmen.

US 4 611 288 offenbart eine Vorrichtung gemäss Oberbegriff von Anspruch 1.

### Darstellung der Erfindung

Es stellt sich somit die Aufgabe, ein Verfahren und eine Messvorrichtung der eingangs genannten Art bereitzustellen, welche eine effiziente lückenlose Ausmessung des Mundraums, und insbesondere einer Zahnreihe oder Teile einer Zahnreihe, erlauben. Der Begriff Zahnreihe bezeichnet hierbei den bezahnten, den teilbezahnten sowie den zahnlosen Kieferkamm inklusive Gaumen. Die Messung soll über einen Teil des Kiefers, insbesondere mindestens einen Zahn, über einen halben oder einen ganzen Kiefer durchgeführt werden können und soll auch den Schlussbiss und die Artikulationsbewegungen des Unterkiefers in Relation zum Oberkiefer erfassen können.

Diese Aufgabe wird vom Gegenstand der unabhängigen Ansprüche gelöst.

Gemäss einen ersten Aspekt betrifft die Erfindung somit eine Messvorrichtung zum Ausmessen mindestens eines Teils eines Mundraums, insbesondere mindestens eines Teils einer Zahnreihe, mit einem Messkopf. Der Messkopf weist dabei zumindest folgende Elemente auf:
- mindestens zwei Messeinheiten, wobei jede Messeinheit einen Messbereich besitzt und wobei die Messbereiche so anordenbar sind, dass sie unterschiedliche Areale des Teils einer Zahnreihe erfassen, und
- einen Träger, welcher die Messeinheiten trägt.

Anspruchsgemäss ist dabei die relative Anordnung der Messbereiche mindestens zweier der erwähnten Messeinheiten verstellbar. Die Erfindung umfasst dabei mehrere Möglichkeiten der Verstellung, insbesondere: a) durch Veränderung der Form des Trägers, z.B. kann durch Deformation des Trägers oder durch Bewegungsmechaniken/Scharniere die relative Anordnung der Messeinheiten zueinander verstellt werden, womit auch die Anordnungen der Messbereiche geändert werden; b) durch Bewegung der Messeinheiten selbst, z.B. mittels Aktuatoren etc, auf dem sonst unveränderten Träger lassen sich die Messbereiche ebenfalls verstellen und c) durch Veränderung des Messbereichs einzelner Messeinheiten, z.B. durch die Änderung der Position eines Spiegels, bei ansonsten unveränderter Ausrichtung der Messeinheiten.

Bei der beanspruchten "Vermessung" handelt es sich vorzugsweise um eine dreidimensionale Vermessung der Oberfläche.

Allerdings können auch Farbinformationen miterfasst werden. Beispielsweise können über die 3D-Oberfläche noch die Farbbilder in Form einer Textur überlagert werden, um und so eine fotorealistische Wiedergabe der intraoralen Situation zu ermöglichen. Diese bevorzugte Ausführung ist auch immer in der weiteren Beschreibung und in den Ansprüchen vor Augen zu haben.

Weiter können durch die Messeinheiten zusätzlich zur dreidimensionalen Erfassung der Oberfläche auch Messungen in die Tiefe der Strukturen durchgeführt werden. Hierzu können zum Beispiel OCT (optische Kohärenztomographie) und/oder Ultraschalltechniken eingesetzt werden, die neben der 3D-Oberfläche auch noch Schichten unterhalb der 3D-Oberfläche erfassen und so weitere Informationen über das Objekt liefern können. Verfahren zur Ultraschallmesstechnik sind z.B. beschrieben in Vollborn et al., 2014 (Ref. 1) und Verfahren im dentalen Bereich zur OCT in Chen et al., 2012 (Ref. 2), Hsieh et al., 2013 (Ref. 3) und Malchow, 2014 (Ref. 4).

Die Vorrichtung ist so ausgestaltet, dass mindestens ein Teil des Mundraums damit ausgemessen werden kann. Darunter sind insbesondere Teile eines Kiefers und/oder der umgebenden Strukturen zu verstehen, insbesondere Teile einer Zahnreihe. So können z.B. Zahnoberflächen, einzelne Zähne, Zähne mit umliegenden Gewebe (feste und bewegliche Schleimhaut), Gebissabschnitte mit mehreren Zähnen oder die gesamte Zahnreihe im Oberkiefer und Unterkiefer vermessen werden. Weiter kann z.B. auch der Gaumen ausgemessen werden, und/oder es können Teile des Ober- und Unterkiefers gleichzeitig erfasst werden, z.B. in Form einer Bukkalaufnahme oder einer Folge von Bukkalaufnahmen während der Artikulation.

Dank der Erfindung können auch Bereiche erfasst werden, die mit herkömmlichen Verfahren nur schwer zu vermessen sind, z.B. Approximalbereiche zwischen zwei Zähnen oder zwischen Präparation und Nachbarzähnen, Kavitäten komplexer Präparationen, wie sie z.B. für Füllungen/ Inlays/ Onlays /Teilkronen und Endokronen erfolgen müssen, Schleimhautbereiche und Lücken zwischen Zähnen und allgemein zahnfreie Schleimhautoberflächen inklusive des gesamten Gaumenareals.

Unter einer "Messeinheit" (auch als "Messmittel" bezeichnet) ist eine Vermessungskomponente zu verstehen, die in der Lage ist, zumindest eine Oberfläche in einem Messbereich dreidimensional zu erfassen. Dabei können alle Varianten z.B. der 3D-Vermessung, wie Stereophotogrammetrie, strukturierte Beleuchtung, konfokale Verfahren, interferometrische Verfahren, wie Weisslicht oder Speckleinterferometrie, optische Kohärenz-Tomographie (OCT) (Ref. 2 - 4) und auch Ultraschall 3D-Scanning (Ref. 1) in Frage kommen.

Eine Messeinheit kann zum Beispiel eine Kamera mit Kameraoptik und eine Beleuchtung mit strukturierter Projektionsoptik aufweisen, um eine 3D-Vermessung durchzuführen. Ebenso sei als Beispiel für eine Messeinheit die Stereoanordnung von zwei Kameras erwähnt. Als Messeinheit kann auch ein Baustein basierend auf einem konfokalen, interferometrischen oder OCT-Verfahren dienen.

Für die Findung von Korrespondenzen in den versetzten Bildern können dann Eigenstrukturen der Oberfläche analysiert werden, die sich z.B. aus einer Aufnahme unter Beleuchtung mit weissen LED-Licht ergeben, aber auch aus einer Folge von Aufnahmen mit LEDs unterschiedlicher Wellenlänge, die der Reihe nach angesteuert werden.

Um die Messzeiten kurz zu halten, damit keine Verwacklungen während der Vermessung auftreten, können auch Hochgeschwindigkeitskameras eingesetzt werden.

Vorzugsweise ist die Messeinheit miniaturisiert, wodurch eine kompakte intraorale räumliche Anordnung und hohe Flexibilität ermöglicht wird. Als Beispiel seien hier Kameraarrays und Projektorarrays sowie Mikrospiegelarrays zu nennen, welche jeweils Arrays von Messeinheiten bilden; weiterhin sind Mikrokameras mit einer Ausdehnung von 1x1x2 mm bekannt (z.B. der Firma Awaiba).

Jede Messeinheit wird durch Parameter wie Tiefenmessbereich, lateraler Messbereich, Auflösung, Aufnahmegeschwindigkeit etc. charakterisiert. Diese Parameter sind an die jeweilige Art der Ausführung der Erfindung anzupassen.

Anspruchsgemäss sind mehrere Messeinheiten auf einem gemeinsamen Träger angeordnet, so dass man z.B. Zähne, grössere Abschnitte von Zahnreihen oder eine ganze Kieferzahnreihe, möglichst von verschiedenen Ansichten, auf einmal vermessen kann. Dabei müssen die Messeinheiten nicht alle identisch sein, sondern es kann z.B. deren Grösse von Messeinheit zu Messeinheit auf dem Träger variieren. Es ist auch denkbar, Messeinheiten mit unterschiedlichen Messverfahren auf dem Träger zu kombinieren.

Anspruchsgemäss ist die relative Anordnung der Messebereiche mindestens zweier Messeinheiten verstellbar. Unter "Anordnung" ist dabei die Position und/oder die Ausrichtung der Messbereiche zu verstehen. Mit anderen Worten können also die Messbereiche der Messeinheiten relativ zueinander in verschiedene Positionen und/oder Ausrichtungen gebracht und so den Messerfordernissen angepasst werden.

Der Träger kann mit Vorteil mindestens in eine erste und eine zweite Konfiguration gebracht werden, wobei die relative Anordnung der Messbereiche von mindestens zwei der Messeinheiten sich in der ersten und der zweiten Konfiguration unterscheidet. Durch Wahl der Konfiguration des Trägers können die Anordnungen der Messbereiche an die zu messende Oberfläche angepasst werden. Somit kann der Träger vor oder während dem Messprozess an die morphologischen Verhältnisse angepasst werden.

Der Träger kann beispielsweise mindestens zwei beweglich miteinander verbundene Messkörper aufweisen, wobei an jedem Messkörper mindestens eine Messeinheit angeordnet ist. Durch Verstellen der Messkörper zueinander kann die Anordnung der Messbereiche variiert werden.

Der Träger kann auch mindestens teilweise aus einem plastisch oder elastisch deformierbaren Material bestehen, mit welchem er reversibel zumindest von der ersten in die zweite Konfiguration und von der zweiten in die erste Konfiguration übergeführt werden kann. Beispielsweise kann der Träger wie ein gummiähnliches Material elastisch dehnbar sein, wobei die Messeinheiten in dieses eingearbeitet sind. Als Träger kommt auch eine Folie infrage, die von Hand geformt und während des Messprozesses gehalten wird. Als analoges Beispiel sei hierzu die Registrierung der Zahnkontakte beim Kieferschluss mithilfe der T-Scan (Ref. 7, 8) Sensorfolie genannt.

Vorzugsweise weist der Träger Mikrogelenke oder Scharnierachsen auf, die eine grosse Flexibiliät und Anpassbarkeit ermöglichen. Die Scharniere können mit Federn für eine elastische Rückstellung, mit einem Rastmechanismus zum Einrasten in bestimmten Positionen und/oder mit Mikromotoren für eine automatische Justierung versehen sein.

Vorteilhaft ist also vorgesehen, dass die Messeinheiten miteinander oder gegeneinander bewegt werden können, sei es direkt über Kippbewegungen der Messeinheiten oder Teile der Messeinheiten selbst oder indirekt über eine Konfigurierbarkeit des Trägers. Es ist keine feste Beziehung bezüglich Ausrichtung oder Messraum zwischen den einzelnen Messeinheiten notwendig.

Anstatt die gesamte Messeinheit zu bewegen, kann auch nur der Strahlengang der Messeinheiten über Spiegel, insbesondere über Mikrospiegelarrays, verändert werden (Ref. 9). Es können auch andere Komponenten der Messeinheiten verstellbar ausgestaltet sein, soweit diese Komponenten eine Änderung des Strahlenganges und damit des Messbereichs ermöglichen.

Im Vergleich zu konventionellen Techniken besteht damit die Möglichkeit, individuell unterschiedliche Kieferabschnitte, tiefe Kavitäten, Approximalbereiche oder auch Schleimhautabschnitte sowohl mit derselben Vorrichtung als auch in einem einzigen Messprozess zu erfassen, was das Einsatzgebiet und die Benutzerfreundlichkeit deutlich erhöht.

Die Messeinheiten erfassen unterschiedliche Abschnitte der auszumessenden Oberfläche. Es ist von Vorteil, wenn sich die Messbereiche der Messeinheiten teilweise überlappen. Allerdings ist dies keine notwendige Voraussetzung. Die Erfassung der gesamten Oberfläche kann auch durch Bewegung oder Verschiebung der gesamten Vorrichtung oder durch Kippen der einzelnen Messeinheiten erfolgen. Die dabei erzeugten einzelnen 3D-Teilaufnahmen der Oberfläche können z.B. anschliessend durch Verfahren wie das globale Registrieren zu einer Gesamtansicht zusammengelagert werden (Krishnan et al 2005, Ref. 5; Fisher und McDonagh 2013, Ref 6). Dabei muss die gegenseitige räumliche Zuordnung der einzelnen Ansichten a priori nicht bekannt sein.

Bei der Analyse der Aufnahmen kann auch detektiert werden, ob gewisse Areale der Oberfläche noch nicht erfasst worden sind. Falls dies der Fall ist, kann dem Benutzer Feedback gegeben werden, worauf dieser die Aufnahme in dem gewissen Areal nachholen kann.

In einer weiteren vorzugsweisen Ausführung besitzt die Messvorrichtung einen Schaft für den Zugang in die Mundhöhle, an dessen Ende ein Messkopf angebracht ist. Der Messkopf ist vorteilhaft beweglich mit dem Schaft verbunden.

Der Messkopf bildet den erwähnten Träger und trägt die Messeinheiten.

Vorteilhaft besitzt der Messkopf mindestens zwei, insbesondere drei oder mehr Messkörper, welche zueinander und in Bezug auf den Schaft beweglich sind.

Der Träger kann zumindest teilweise auch aus einem formbaren, sowie dehn- und kontrahierbaren Material mit Rückstellungseigenschaften bestehen, auf dem die Messeinheiten bezüglich ihrer Winkelorientierung gegenüber der Zahnreihe und bezüglich ihrer Distanz voneinander in den Raumachsen X, Y und Z bedarfsgerecht orientiert werden können.

Anspruchsgemäss besteht also zwischen den Messbereichen der Messeinheiten keine feste räumliche Beziehung. Eine solche Vorrichtung kann sich etnweder automatisch oder durch manuelle Manipulation an beliebige Topologien der Zahnreihe anpassen.

Beispielsweise können ein mit dem Schaft verbundener zentraler Messkörper sowie zwei beweglich am zentralen Messkörper angeordnete seitliche Messkörper den Träger bilden. Die Konfiguration des Trägers kann in diesem Falle durch Schwenken der seitlichen Messkörper gegenüber dem zentralen Messkörper variiert werden. Dies erlaubt es, den Messkopf den unterschiedlichen Kiefertopologien anzupassen und die Zahnreihe aus unterschiedlichen und variablen Raumwinkeln zu vermessen.

Die im zentralen und in den beiden seitlichen Messkörpern angeordneten Messmittel dienen dazu, einen im Messraum angeordneten Abschnitt der Zahnreihe dreidimensional auszumessen

Besitzt die Messvorrichtung einen Schaft, so kann dieser fest oder beweglich mit dem zentralen Messkörper verbunden sein. Mit Hilfe des Schaftes kann der Messkopf entlang der Zahnreihe im Kontakt mit der Zahnreihe oder kontaktfrei geführt werden.

Der Messkopf definiert einen Messraum, in welchem die Strukturen des Gaumens ausgemessen werden können. Vorteilhaft ist dieser Messraum entlang einer Achse X beidseitig offen. Zumindest in einer Konfiguration des Trägers wird der Messraum entlang einer Achse Y beidseitig vom Messkopf begrenzt und ist entlang einer Achse Z einseitig offen und einseitig vom Messkopf begrenzt. Dabei stehen die erwähnten Achsen X, Y und Z senkrecht aufeinander.

Wenn der Träger aus den erwähnten seitlichen Messkörpern und dem zentralen Messkörper besteht, so begrenzen die seitlichen Messkörper in der erwähnten Konfiguration den Träger beidseits in Richtung der Achse Y, und der zentrale Messkörper begrenzt den Messraum einseitig in Richtung der Achse Z.

Vorteilhaft weist der Messkopf folgende Elemente auf:
mindestens zwei seitliche Führungskörper mit Führungsbereichen zum seitlichen Anlegen an die Zahnreihe und
mindestens zwei seitliche Messkörper, wobei wenigstens ein Teil der Messeinheiten in den seitlichen Messkörpern angeordnet ist;
vorzugsweise sind dabei die seitlichen Führungskörper gegeneinander derart beweglich, dass der Abstand der Führungsbereiche der seitlichen Führungskörper voneinander um mindestens 3 mm variierbar ist, und insbesondere wobei auch die seitlichen Messkörper gegeneinander beweglich sind.

Vorzugsweise erstrecken sich die seitlichen Messkörper entlang der Achse X über eine Länge von 3 bis 12 mm, insbesondere 4 bis 8 mm, und/oder die seitlichen Führungskörper entlang der Achse X über eine Länge von 4 bis 30 mm, insbesondere 6 bis 16 mm.

Vorteilhaft ist in diesem Falle der Messkopf so konfiguriert oder konfigurierbar, dass entlang der Achse Z der Abstand der Führungsbereiche der Führungskörper von einem den Messbereich entlang Achse Z begrenzenden Teil des Messkörpers zwischen 5 und 20 mm liegt und entlang der Achse Y der Abstand (Dy) der Führungsbereiche der seitlichen Führungskörper voneinander zwischen 10 und 24 mm, insbesondere 14 und 22 mm, liegt.

In einer weiteren bevorzugten Ausführung sind die seitlichen Messkörper und/oder die seitlichen Führungskörper zueinander verschwenkbar angeordnet, und insbesondere sind die seitlichen Messkörper und/oder die seitlichen Führungskörper zueinander um mindestens 30°, insbesondere mindestens 90°, schwenkbar, um so den Messraum flexibel an die jeweiligen Anforderungen anzupassen.

In einem weiteren Aspekt betrifft die Erfindung eine Messvorrichtung, insbesondere der oben beschriebenen Art, zum Ausmessen mindestens eines Teils einer Zahnreihe mit
einem Messkopf, welcher (in mindestens einer der Konfigurationen) einen Messraum definiert, der entlang einer Achse X beidseitig offen ist, entlang einer Achse Y beidseitig vom Messkopf begrenzt ist und entlang einer Achse Z einseitig offen und einseitig vom Messkopf begrenzt ist, wobei die Achsen X, Y und Z senkrecht zueinander angeordnet sind und

Messeinheiten, um einen im Messraum angeordneten Bereich der Zahnreihe dreidimensional auszumessen,
wobei der Messkopf aufweist:
mindestens zwei seitliche Führungskörper mit Führungsbereichen zum seitlichen Anlegen an die Zahnreihe und
mindestens zwei seitliche Messkörper, wobei wenigstens ein Teil der Messeinheiten in den seitlichen Messkörpern angeordnet ist.

Vorteilhaft ist der Messkopf so konfiguriert oder konfigurierbar (d.h. in der mindestens einen Konfiguration derart gestaltet), dass entlang der Achse Z der Abstand der Führungsbereiche von einem den Messbereich entlang Achse Z begrenzenden Teil des Messkörpers zwischen 5 und 20 mm liegt und entlang der Achse Y der Abstand der Führungsbereiche der seitlichen Führungskörper voneinander zwischen 10 und 24 mm, insbesondere 14 und 22 mm, liegt.

In einer weiteren bevorzugten Ausführung der beiden ersten Aspekte der Erfindung besitzt die Messvorrichtung ein Auflager geeignet zum Abstützen des Messkopfs auf einer Zahnreihe sowie einen Kippaktuator, mit welchem der Träger gegenüber dem Auflager automatisiert (d.h. elektronisch gesteuert) verkippt werden kann. Dies erlaubt es, den Träger während einer Messung zu bewegen, wodurch mehr Aufnahmen oder Aufnahmen aus besseren Blickwinkeln erstellt werden können. Vorteilhaft ist das Auflager fest mit einem Schaft der Messvorrichtung verbunden, mit welchem der Benutzer die Messvorrichtung führen kann.

In einem weiteren Aspekt betrifft die Erfindung ein Verfahren zum Ausmessen mindestens eines Teils eines Mundraums, insbesondere mindestens eines Teils einer Zahnreihe mit einer Messvorrichtung der oben beschriebenen Art. Das Verfahren umfasst die folgenden Schritte:
a) Positionieren des Messkopfs derart über einer Struktur des Mundraums, insbesondere über einer Zahnreihe, dass ein Abschnitt der Struktur im Messraum zu liegen kommt, und insbesondere wobei die Messkörper mindestens mit ihren seitlichen Führungskörpern mit der Zahnreihe in Berührung kommen, und
b) Führen des Messkopfs entlang der Struktur, insbesondere derart, dass mindestens ein seitlicher Führungskörper mit der Zahnreihe in Berührung bleibt,
c) Ausmessen der Struktur, insbesondere während dem Führen des Messkopfs.

Vorteilhaft besitzt die Messvorrichtung einen Schaft, an welchem der Benutzer die Messvorrichtung während dem Ausmessen der Zahnreihe hält. Weiter besitzt sie einen elektronisch gesteuerten Kippaktuator, um den Messkopf um mindestens eine Achse gegenüber dem Schaft zu kippen. In diesem Falle kann während dem Ausmessen der Struktur der Messkopf gegenüber dem Schaft der Messvorrichtung mit dem Kippaktuator in unterschiedliche Stellungen gekippt werden, um Messaufnahmen aus unterschiedlichen Blickwinkelpositionen zu erzeugen.

Weiter kann die Konfiguration des Trägers beim Führen des Messkopfs entlang der Struktur variiert werden, um so den Messbereich an die lokale Konfiguration der auszumessenden Struktur anzupassen. Wenn der Messkopf, wie oben erwähnt, einen zentralen Führungskörper und mindestens zwei schwenkbar am zentralen Führungskörper angeordnete seitliche Messkörper aufweist, so kann die Winkelstellung der seitlichen Messkörper gegenüber dem zentralen Messkörper beim Führen des Messkopfs entlang der Struktur variiert werden.

Vorzugsweise wird dabei die Winkelstellung der seitlichen Messkörper gegenüber dem zentralen Messkörper beim Führen des Messkopfs entlang der Struktur variiert. Insbesondere kann während der Führung des Messkopfs entlang der Struktur, wenn Kontakt zwischen mindestens einem der Führungskörper des Messkopfes und der Struktur besteht, die Position der seitlichen Messkörper z.B. durch Signale von Kontakt- oder Distanzsensoren in den Führungskörpern der seitlichen Messkörper und Signale von Winkelsensoren in den Scharnieren zwischen dem zentralen und den seitlichen Messkörpern durch Mikromotoren, automatisch der Breite der Struktur angepasst werden.

Im Rahmen der Führung des Messkopfs entlang der Struktur kann durch voreingestellte unterschiedliche Winkelpositionen der Messmittel gegenüber dem Messkopf und/oder durch eine automatisch gesteuerten Raumwinkeländerungen des ganzen Messkopfes mittels Kippaktuator, eine lückenlose dreidimensionale Vermessung aus unterschiedlichen Raumwinkeln ermöglicht werden.

Um Messaufnahmen aus unterschiedlichen Blickwinkelpositionen ohne manuelle Richtungsänderungen am Schaft zu erzeugen und so eine höhere Genauigkeit und einen erweiterten Messraum zu erhalten, kann während dem Ausmessen der Struktur mindestens ein Teil der Messeinheiten gegenüber den Wänden der Messkörper in unterschiedlichen Winkel-Stellungen fest ausgerichtet sein, oder in unterschiedliche Winkelpositionen bewegt werden, oder unterschiedliche Blickwinkel können auch durch gesteuerte Bewegungen des von den Messeinheiten erzeugten Strahlenganges mittels Spiegeln eingestellt werden, oder der ganze Messkopf kann mindestens in einer der Achsen X, Y oder Z aktiv gekippt werden, um Messaufnahmen aus unterschiedlichen Raumwinkelpositionen zu erzeugen. Das Kippen des ganzen Messkopfes kann insbesondere mittels elektronisch gesteuerten Kippaktuatoren und im Speziellen automatisch erfolgen.

Die Führung des Messkopfs entlang der Struktur kann kontinuierlich mit kontrollierter Geschwindigkeit oder abschnittsweise erfolgen. Für eine kontinuierliche Führung weist die Messvorrichtung vorteilhaft Ausgabemittel auf, z.B. in Form von optischen oder akustischen Signalgebern, welche angeben, ob die Geschwindigkeit der Führung korrekt ist. Bei einer intervallweisen, d.h. abschnittweisen Führung weist die Messvorrichtung vorteilhaft Ausgabemittel auf, welche angeben, wann der Messkopf zu bewegen ist, d.h. wann die nächsten Aufnahmen erfolgen können.

Zum Ausmessen einer Zahnreihe kann der Messkopf vorteilhaft mit einem entlang der Achse X angebrachten Schaft ohne Absetzen (d.h. ohne dass er von der Zahnreihe abgenommen wird) entlang der Zahnreihe einer Kieferhälfte bis über die Kiefermitte geführt werden. In einer anderen vorteilhaften Ausführung kann der Messkopf ohne Absetzen über die Zahnreihe des ganzen Kiefers geführt werden, wenn der Schaft auf der Oberseite des zentralen Messkörpers um die Achse Y entlang der Achse X schwenkbar mit dem Messkopf verbunden ist. Dabei wird der Schaft jeweils beim Durchgang durch die Kiefermitte über die Achse Y geschlagen.

In einer Ausführung kann der Messkopf, wie bereits ausgeführt, einen zentralen, kauflächenseitigen Messkörper aufweisen, welcher dazu geeignet ist, mit seiner dem Messraum zugewandten Seite an die Zahnreihe angelegt zu werden. Dabei begrenzt der zentrale Messkörper den Messraum entlang der Achse Z und erlaubt es, den Messkopf in dieser Richtung in eine definierte Position zu bringen. Ist der zentrale Messkörper entlang der Achse X starr oder über ein Kippaktuator mit dem Schaft verbunden, so kann die Positionierung des Messkopfes in der Achse Z durch die Höhe des Auflagers an der Unterseite des Schaftes definiert werden.

Die seitlichen Messkörper können seitlich schwenkbar oder flexibel am zentralen Messkörper angeordnet werden.

Vorteilhaft sind beide seitlichen Messkörper an ihren freien Kanten, d.h. den dem zentralen Messkörper gegenüber liegenden, sich entlang der Achse X erstreckenden Kanten, mit einem flexiblen Führungskörper aus Weichplastik besetzt, der zur Zahnreihe vorragt und den Kontakt mit der Zahnreihe herstellen kann. Dieser seitliche Führungskörper kann einen Mindest-Messabstand des seitlichen Messkörpers von der Zahnreihe begrenzen.

Vorteilhaft besitzt der Messkopf mindestens einen Kontakt- oder Distanzsensor, um einen Kontakt des Messkopfs mit dem auszumessenden Mundraum zu detektieren, d.h. um zu detektieren, ob der Messkopf die Schleimhaut oder eine Zahnstruktur berührt. In diesem Fall ist die Messvorrichtung derart ausgestaltet, dass die die Konfiguration des Trägers abhängig vom Signal des Kontakt- oder Distanzsensors variiert, womit die Konfiguration der Topologie der zu messenden Struktur angepasst werden kann.

Beispielsweise kann hierzu an der Berührungsfläche zur Zahnreihe im seitlichen Führungskörper mindestens ein Kontakt- oder Distanzsensor untergebracht sein, dessen Signale zur Winkelsteuerung des seitlichen Messkörpers dienen können. Dabei kann die Winkelbewegung, durch in Scharnieren entlang der Achse X eingebaute Mikromotoren, angetrieben und/oder durch Winkelsensoren im Scharnier kontrolliert werden.

Vorteilhaft sind in den seitlichen Messkörpern mindestens ein Teil der Messmittel optische Sensoren zum dreidimensionalen Ausmessen des sich im Messbereich befindlichen Abschnitts der Zahnreihe vorgesehen. Ein Teil dieser optischen Sensoren ist im zentralen Messkörper angeordnet. Auf diese Weise kann die Zahnreihe von der Kau-Ebene her, aber auch seitlich gut erfasst werden.

Vorteilhaft weist der Messkopf mindestens eine Echtfarbenkamera zum Erstellen von Aufnahmen in Echtfarben auf. Insbesondere weisen alle Messkörper mindestens eine Echtfarbenkamera. Die Echtfarbaufnahmen können für das Rendering der 3D Oberflächendarstellung, zur klinischen Dokumentation aber auch zur Ermittlung von Farbwerten für die Farbwahl der Restaurationsmaterialien verwendet werden. Die Echtfarbenkamera kann Teil der Messeinheiten zur dreidimensionalen Ausmessung der Zahnreihe sein, oder sie kann als separates zusätzliches Bauteil vorgesehen werden.

Um eine Anpassung des Messkopfes an die unterschiedliche Breite der Zahnreihen bzw. der unbezahnten Kieferkämme zu realisieren, sowie den Messbereich für die Erfassung der Gaumenoberfläche, des Schlussbisses sowie der der Artikulationsbewegungen des Unterkiefers und zu erweitern, können die Messkörper in einer einfachen Ausführung zueinander schwenkbar angeordnet sein, z.B. durch Scharniere oder elastische oder plastische Deformation eines Verbindungsmaterials zwischen den Messkörpern, wie z. B. Elastomere oder Metalle mit Rückstelleigenschaft, sie können z.B. aber auch zusätzlich translatorisch zueinander beweglich ausgestaltet sein. Es ist auch möglich, dass einzelne Messeinheiten in den Messkörpern in unterschiedliche Raumwinkel gekippt oder translatorisch bewegt werden.

Zur Kontrolle und Steuerung der Winkelstellung der Messkörper gegeneinander sind vorteilhaft Winkelsensoren vorgesehen, mit welchen die Winkelposition der seitlichen Messkörper in Relation zum zentralen Messkörper erfasst werden kann. Durch Berücksichtigung der Winkelposition können die Messungen, welche von den Messmitteln in den unterschiedlichen Teilen des Messkopfs durchgeführt werden, besser miteinander korreliert werden.

Die seitlichen Messkörper sind von ihrer Standardposition mit 90° Innenwinkel zum zentralen Messkörper vorteilhaft um mindestens 20° nach innen und mindestens 90° nach aussen schwenkbar, insbesondere um eine Schwenkachse, welche entlang der Achse X verläuft.

In einer besonders bevorzugten Ausführung, in welcher die seitlichen Messkörper schwenkbar an einem zentralen Messkörper angeordnet sind, können die seitlichen Messkörper zumindest zwischen einer ersten Position, in welcher die dem Messraum zugewandten Innenwinkel höchstens 90° betragen, und einer zweiten Position im Bereich von 135° - 170° geschwenkt werden. Diese Stellung der seitlichen Messkörper erlaubt es, den Messkopf im Mundvorhof (Vestibulum), auf die Mundschleimhaut der Alveolarfortsätze der geschlossenen Ober- und Unterkiefer-Zahnreihen aufzusetzen und den Schlussbiss sowie die Seitbewegungen des Unterkiefers gegen den Oberkiefer zu vermessen. Die Führungskörper am freien Ende der seitlichen Messkörper können in dieser Position als Stützen für den zentralen Messkörper dienen, der durch die Winkelstellung der seitlichen Messkörper mit seinen Messmitteln in einen optimalen Messabstand gebracht und mit Hilfe des Führungsschaftes stabilisiert werden kann. Diese Art von Messung erlaubt es, die Messvorrichtung auch für die räumliche Vermessung des Schlussbisses beider Kiefer und für die Aufzeichnung der funktionellen Bewegungen des Unterkiefers zu verwenden.

Besonders vorteilhaft kann die Beweglichkeit der seitlichen Messkörper für die Ausmessung des Gaumens eingesetzt werden, wenn die seitlichen Messkörper gegenüber zentralen Messkörpers vorzugsweise soweit verschwenkt werden, dass die Messraumseitigen Winkel α mindestens 200°, beispielsweise 250°, betragen. Dadurch entsteht ein Messraum, der mithilfe der Führungskörper der seitlichen Messkörper an das Gaumengewölbe angepasst werden kann und welche die Voraussetzung für eine schnelle und vollständige Vermessung des Gaumens schafft.

Besonders vorteilhaft kann ein Antrieb für die Winkeleinstellung zwischen den Messkörpern durch je einen in den beiden seitlichen Scharnierachsen angeordneten Mikromotor erfolgen. Durch die Mikromotoren können auch vordefinierte Winkelpositionen angefahren werden.

Besonders vorteilhaft kann, wie erwähnt, die mikromotorgetriebene Winkelstellung der seitlichen Messkörper durch Kontakt- oder Distanzsensoren gesteuert werden, die in den Führungskörpern der seitlichen Messkörper angeordnet sind. Bei Einsatz der Messvorrichtung können die Winkelstellungen der seitlichen Messkörper einzeln so gesteuert werden, dass Spielraum für die Kippbewegungen des Messkopfes berücksichtigt wird. Die Winkelstellungen der seitlichen Messkörper können auf diese Weise beim Scannen entlang der Zahnreihe automatisch den unterschiedlichen Querschnitten der Zahnreihe bzw. zahnloser Kieferkämme und dem Gaumengewölbe angepasst werden.

Vorteilhaft sind mit einem auf der Oberseite des Messkopfes am zentralen Messkörper beweglich angebrachten Schaft Kontaktsensoren im Verbindungselement zwischen dem Schaft und dem zentralen Messkörper vorgesehen. Ist in einer anderen Ausführung der Schaft mit dem zentralen Messkörper entlang der Achse X verbunden, können Sensoren im Auflager des Schaftes den Kontakt mit der Zahnreihe messen. Das Auflager kann die Unterseite des Schaftes im Bereich von 14 - 60 mm abdecken. Vorteilhaft kontrollieren die Kontaktsensoren, ob die Vorrichtung abgestützt ist. Besteht im Falle einer ausgedehnten Zahnlücke kein Kontakt, so kann für die kontaktfreie Messung die Winkelstellung der seitlichen Messkörper in eine Standardposition gefahren werden.

Besonders vorteilhaft kann das dreidimensionale Ausmessen der Zahnreihe durch eine bewegliche Verbindung des Messkopfes mit dem Schaft über einen Kippaktuator entlang der Achse X ausgeführt werden. Der Messkopf kann gegenüber dem Schaft und dessen Auflager auf der Zahnreihe beweglich gehalten werden, insbesondere mithilfe des erwähnten Kippaktuators. Die Höhe des Auflagers bestimmt dabei den Abstand des zentralen Messkörpers von der Zahnreihe. Beispielsweise erlauben Kippbewegungen um die Y- und Z-Achsen, die gesamten Messmittel des Messkopfes in eine gesteuerte Sequenz von unterschiedlichen Blickwinkelpositionen zu bringen, wobei in diesen Positionen Messaufnahmen erfolgen können. Der Kippaktuator kann Piezoelemente aufweisen. Diese können im Schaft untergebracht werden.

Zum Führen der Messvorrichtung kann am Messkopf ein mindestens 40 mm, insbesondere mindestens 100 mm langer Schaft vorgesehen sein.

Im Schaft können, wie erwähnt, die Piezo-Elemente des Kippaktuators mit genügender Länge für grosse Kippwinkel untergebracht werden. Beispielsweise können vier Piezo-Elemente kreuzweise angeordnet werden, wodurch Kippbewegungen um zwei senkrechte Achsen, insbesondere die Achsen Y und Z, erzeugt werden können. Die Kippwinkel können insbesondere zwischen 0.25 - 12°, vorzugsweise 0.5 - 6° liegen.

Um eine gute Führung des Messkopfs auf der Zahnreihe zu gewährleisten und die Möglichkeit zu haben, den Messkopf über die am stärksten gebogenen Bereiche der Zahnreihe zu führen, erstrecken sich die seitlichen Führungskörper in Richtung der Achse X vorzugsweise über eine Länge von 4 - 30 mm, insbesondere über eine Länge von 6 - 16 mm.

Die Messvorrichtung kann grundsätzlich zum intraoralen Ausmessen irgendeiner Struktur oder Bewegung im Mundraum verwendet werden. Vorzugsweise ist die Struktur bzw. Bewegung mindestens ein Teil einer Zahnreihe, mindestens ein Teil eines Kieferkamms, ein Schlussbiss, die Bewegung des Unterkiefers oder mindestens ein Teil eines Gaumens.

Die Messvorrichtung kann mindestens in den Scharnierbereichen zwischen den Messkörpern durch die Abdeckung mit einer Elastomerschicht bakteriendicht verkleidet werden, womit sie nach dem Einsatz am Patienten effizient desinfiziert werden kann.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine schematische Skizze, in perspektivischer Seitenansicht, eines Messkopfes mit einem zentralem und zwei schwenkbaren seitlichen Messkörpern, wobei der Messkopf über ein Kippaktuator entlang der Achse X beweglich mit einem Schaft verbunden ist, über einer Zahnreihe,
Fig. 2 eine schematische Schnittdarstellung des Messkopfes nach Fig.1 über einem Seitenzahn mit einem zentralen und zwei schwenkbaren seitlichen Messkörpern,
Fig. 3 schematische Schnittdarstellungen von Messanordnungen mit einem formbaren Träger, der in a) und d) über einem Seitenzahn, in b) und e) auf der Bukkalseite bei Schlussbiss und Artikulation und in c) und f) im Gaumengewölbe der Topologie angepasst ist, wobei a) bis c) Beispiele für Ausführungen von Messeinheiten mit Projektoren und Mikrokameras darstellen, während d) bis f) Beispiele für Ausführungen mit beliebigen Messeinheiten sind,
Fig. 4 eine schematische Skizze, in perspektivischer Seitenansicht, eines Messkopfes mit einem zentralem Messkörper und zwei schwenkbaren seitlichen Messkörpern, mit einem um die Y Achse beweglichem Führungsschaft auf der Oberseite des zentralen Messkörpers,
Fig. 5 eine schematische Schnittdarstellung des Messkopfes nach Fig. 1 über einem Frontzahn mit einer zentralen und zwei nach innen rotierten seitlichen Messkörpereinheiten,
Fig. 6 eine Illustration der Vermessung des Schlussbisses von Ober- und Unterkieferzähnen im Querschnitt mit ausgeschwenkten seitlichen Messkörpern, die den Messkopf auf den Kiefern seitlich abstützen,
Fig. 7. eine Illustration des Messverfahrens in einer QuerschnittsDarstellung des Gaumens mit um 250° nach aussen rotierten seitlichen Messkörpern (α = 250°),
Fig. 8. eine Illustration des Messverfahrens in einer QuerschnittsDarstellung des zahnlosen Unterkiefers mit Abstützung und Führung des Messkopfes auf dem Kieferkamm mit Führungsschaft nach Fig. 3 links und mit Führungsschaft nach Fig. 1, rechts, und
Fig. 9 eine schematische Skizze einer Messeinheit bestehend aus einem Mikroprojektor und einer Mikrokamera mit Mikrospiegeln und einem ersten und einem zweiten Messbereich auf einer Zahnoberfläche.

### Wege zur Ausführung der Erfindung

### Messvorrichtung

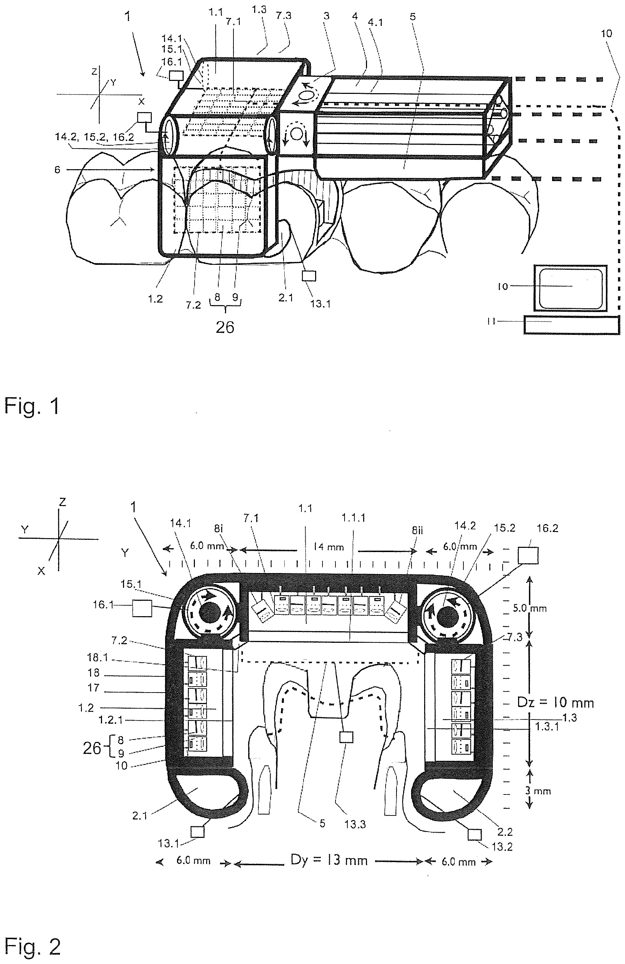

Fig. 1 zeigt die Ausführung einer Messvorrichtung, bei der ein Messkopf 1 über einen Kippaktuator 3 beweglich mit einem Schaft 4 verbundenen ist. Der Messkopf 1 weist einen zentralen Messkörper 1.1 und zwei seitliche, schwenkbar angebrachte Messkörper 1.2, 1.3 auf. Zum Kontakt mit der Zahnreihe besitzen die Messkörper 1.1, 1.2, 1.3 Führungskörper 2.1, 2.2, und der Schaft 4 ist mit einem Auflager 5 zur Abstützung auf der Zahnreihe ausgestattet. Die Vorrichtung ist in perspektivischer Seitenansicht über der Zahnreihe schematisch dargestellt.

Die Führungskörper 2.1, 2.2 sind an den freien Enden der seitlichen Messkörper 1.2, 1.3 angeordnet und erstrecken sich entlang der Achse X.

Die Messkörper 1.1, 1.2, 1.3 bilden zusammen den Träger für die weiter unten beschriebenen Messeinheiten.

Mit dem Kippaktuator 3 kann der Träger 1.1 - 1.3 gegenüber dem Auflager 5 automatisiert verkippt werden.

Der Messkopf 1 definiert aufgrund seiner Orientierung ein Koordinatensystem mit den Achsen X, Y und Z.

In der dargestellten Ausführung nach Fig.1 ist der Messkopf 1 mit seinem zentralen Messkörper 1.1 über den Kippaktuator 3 um die Achsen Y und/oder Z kippbar beweglich am Schaft 4 befestigt. In der Ausführung nach Fig. 1 erstreckt sich der Schaft 4 entlang der Achse X und kann mit dem Auflager 5 auf der Zahnreihe geführt werden. Die Höhe des Auflagers 5 definiert die Distanz des zentralen Messkörpers 1.1 von der Zahnreihe.

Der Messkopf 1 umfasst der Ausführung nach Fig. 1 den parallel zur Kau-Ebene angeordneten zentralen Messkörper 1.1 und die zwei beispielsweise über Scharniere schwenkbar am zentralen Messkörper 1.1 angeordneten seitlichen Messkörper 1.2 und 1.3.

Der Messkopf 1 definiert einen Messraum 6, welcher in Richtung der Achse X beidseits offen ist, in Richtung der Achse Y von den beiden seitlichen Messkörpern 1.2 und 1.3 begrenzt wird und in Richtung der Achse Z vom zentralen Messkörper 1.1 auf einer Seite begrenzt wird und auf der anderen Seite offen ist. Die Messkörper 1.1, 1.2 und 1.3 enthalten Messeinheiten 26, von denen in der Ausführung nach Fig. 1 und 2 jede mindestens einen Mikroprojektor 8 sowie eine Mikrokamera 9 umfasst.

Die seitliche Führung des Messkopfes kann in der vorliegenden Ausführung gemäss Fig.1 durch die seitlichen Führungskörper 2.1, 2.2 gebildet werden, die an den freien Enden der Messkörper 1.2 und 1.3 auf die Zahnreihe gerichtet angebracht sind. Kauebenen-seitig wird die Führung durch das Auflager 5 realisiert.

Die seitlichen Messkörper 1.2, 1.3 erstrecken sich entlang der Achse X beispielsweise über 3 - 20 mm, insbesondere 4 - 17 mm. Diese Maße erlauben, dass die Messvorrichtung leicht durch die innere Kurvatur im Bereich der Eckzähne geführt werden kann.

In den Messkörpern 1.1, 1.2 und 1.3 sind die Messeinheiten 26 (wobei in dieser Ausführung jede einzelne Messeinheit mindestens einen Mikroprojektor 8 und eine Mikrokamera 9 umfasst) angeordnet, mit denen die Zahnreihe im Messraum 6 ausgemessen, d.h. dreidimensional erfasst, werden kann. Die Messeinheiten 26 (jede umfassend einen Mikroprojektor 8 und eine Mikrokamera 9) sind z.B. als Arrays 7.1, 7.2, 7.3 von Mikroprojektoren 8 und Mikrokameras 9 ausgestaltet. Die Messkörper 1.1, 1.2 und 1.3 bilden den Träger für die Messeinheiten 26.

Die Steuerzuleitung 10 zu den einzelnen Messeinheiten 26 werden durch den Messkopf 1 geführt und gelangen durch den Kippaktuator 3 und den Schaft 4 zu einem Steuergerät 11, wo auf einem Monitor 12 die zusammengeführten Einzelaufnahmen der Messeinheiten 26 als Gesamtaufnahme der Zahnreihe dargestellt werden können.

Der Schaft 4 bildet die Fortsetzung des zentralen Messkörpers 1.1 in Richtung der Achse X und kann diesen in der Länge um mindestens 40 mm ergänzen. Der Schaft kann beispielsweise zwischen 40 und 150 mm lang sein, eine Gesamthöhe (in Richtung der Achse Z) von 5 - 30 mm und eine Breite (in Richtung der Achse Y) von 10 - 30 mm aufweisen. Dies erlaubt es, lange Piezo-Elemente 4.1 im Schaft unterzubringen, die als Aktuatoren definierte Kippbewegungen des Kippaktuatores 3 mindestens um die Achsen Y und Z bewirken können. Dadurch kann der gesamte Messkopf in eine gesteuerte Sequenz von unterschiedlichen Blickwinkelpositionen gebracht werden, wobei in diesen Positionen Messaufnahmen erfolgen können.

Durch die Länge der Piezo-Elemente werden relativ grosse Kippwinkel ermöglicht. Beispielsweise können vier Piezo-Elemente 4.1 kreuzweise angeordnet werden, wodurch Kippbewegungen um zwei zueinander senkrecht stehende Achsen, insbesondere die Achsen Y und Z, erzeugt werden können. Die Kippwinkel können insbesondere zwischen 0.25 - 12°, vorzugsweise 0.5 - 6° liegen.

Die Piezoelemente 4.1 erzeugen Kippbewegungen um zwei orthogonale Kippachsen, vorzugsweise die Achsen Y und Z, und können optisch je mindestens 16 mrad Kippwinkel einstellen. Die Piezoelemente 4.1 können mit integrierten Positionssensoren ausgerüstet werden, die eine hohe Positionsstabilität und eine Reproduzierbarkeit im sub-µrad Bereich gewährleisten. Messaufnahmen können in den Endpositionen der Kippungen ausgelöst werden, was vorteilhaft eine gesteuerte sehr feinteilige Vermessung der Zahnreihen erlaubt.

Durch die Gewinnung einer Vielzahl von Bilddaten aus unterschiedlichen Raumwinkeln kann eine hohe Richtigkeit und Präzision der dreidimensionalen Vermessung erzielt werden. □

Die seitlichen Messkörper 1.2 und 1.3 besitzen an ihren freien Enden je ein Stütz- und Führungselement 2.1, 2.2, das während der Messung seitlich an der Zahnreihe bzw. an der Mundschleimhaut anliegen kann. In der Ausführung nach Fig. 1 werden die Stütz-und Führungskörper 2.1, 2.2 aus Elastomer gebildet und können zum Messraum 6 hin die Fläche der Messkörper 1.2 und 1.3 um 1 - 15 mm überragen, um den Messabstand gegenüber der Zahnreihe zu sichern. Die Stütz- und Führungselemente 2.1 und 2.2 können mit Kontakt- oder Distanzsensoren 13.1, 13.2 bestückt sein, wodurch die seitliche Führung des Messkopfes 1 entlang der Zahnreihe und die Winkelsteuerung der seitlichen Messkörper 1.2, 1.3 während der Vermessung gewährleistet werden können. Die Abstände der seitlichen Messkörper 1.2, 1.3 können so gewählt werden, dass die Kippbewegungen beim Scannen nicht beeinträchtigt werden.

Zur Erweiterung des Messraums und zur Anpassung an unterschiedliche Kiefer-Topologien kann die Winkelstellung beider seitlicher Messkörper 1.2 und 1.3 gegenüber dem zentralen Messkörper 1.1 an den Verbindungsstellen beispielsweise durch die Scharniere 14.1, 14,2 mittels mindestens einem Antrieb, insbesondere mittels eingebauter Mikromotoren 15.1 und 15.2, verstellt werden kann. Damit können die seitlichen Messkörper in vordefinierte Positionen geschwenkt werden. Weiter kann der Antrieb mittels Sensoren 13.1, 13.2, 16.1, 16.2, insbesondere Kontakt- oder Distanzsensoren 13.1, 13.2, gesteuert werden, um z.B. die seitlichen Messkörper 1.2, 1.3 in vordefinierten Abständen von einer Kieferreihe zu halten. Die Sensoren können auch von den Messeinheiten 26 (jede einzelne z.B. bestehend aus einen Mikroprojektor 8 und einer Mikrokamera 9) gebildet werden.

Fig. 2 zeigt eine schematische Querschnitts-Skizze der Ausführung des Messkopfes 1 nach Fig. 1 und verdeutlicht seine Dimensionen. Die Vorrichtung kann aussen in Richtung der Achse Y beispielsweise zwischen 18 - 36 mm breit und in Richtung der Achse Z 10 - 30 mm hoch sein.

Die Wände 17 der seitlichen Messkörper 1.2 und 1.3 sowie des zentralen Messkörpers 1.1 können beispielsweise aus Metall ausgebildet und können durch die Scharniere 14.1, 14.2 verbunden sein. Sie bilden den oben erwähnten Träger des Messkopfs 1. Auf ihren Innenflächen können beispielsweise Arrays 7.1, 7.2, 7.3 angeordnet sein, welche aus ca. 1 x 1 Millimeter im Querschnitt messenden und 1,5 mm langen alternierend angeordneten Mikroprojektoren 8 und Mikrokameras 9 aufgebaut sind. Zu diesen führen zu- und abgehende Signalleitungen 10. Gegen den Messraum 6 können die Messkörper durch kratzfestes optisches Glas, beispielsweise Saphirglas verschlossen sein.

Die Einhaltung eines Kauebenen-seitigen Mindest-Messabstandes zwischen der Oberfläche der Messeinheiten 26 und den Oberflächen der Zahnreihen von beispielsweise 1 - 10 mm gewährleistet während der Vermessung das Auflager 5 des Schaftes 4 oder in einer anderen Ausführung ein Führungselement 1.1.1 des zentralen Messkörpers 1.1 aus kratzfestem optischen Glas. Beim 90° Standardwinkel der seitlichen Messkörper 1.2 und 1.3 gegenüber dem zentralen Messkörper 1.1 kann die Zentrierung des Messkopfs 1 durch die Führungskörper 2.1 und 2.2 am freien Ende dieser seitlichen Messkörper 1.2 und 1.3 erfolgen. Beim 90° Standardwinkel der seitlichen Messkörper 1.2 und 1.3 gegenüber dem zentralen Messkörper 1.1 kann die Höhe des gebildeten Messraums 6 zwischen der messraumseitigen Oberfläche 1.1.1 des zentralen Messkörpers 1.1 oder von der Unterseite des Auflagers 5 sowie den freien Enden der Messkörper 1.2 bzw. 1.3 zwischen 6 bis 14 mm betragen, und die lichte Breite kann zwischen 12 - 24 mm, insbesondere 14 - 18 mm, betragen. Die Glaselemente 1.1.1, 1.2.1 und 1.3.1, welche die messraumseitigen Abschlüsse der Messkörper 1.1, 1.2, 1.3 bilden, können einen Mindest-Messabstand zwischen der Oberfläche der Messeinheiten 26 und den Oberflächen der Zahnreihen von beispielsweise 1 - 10 mm gewährleisten.

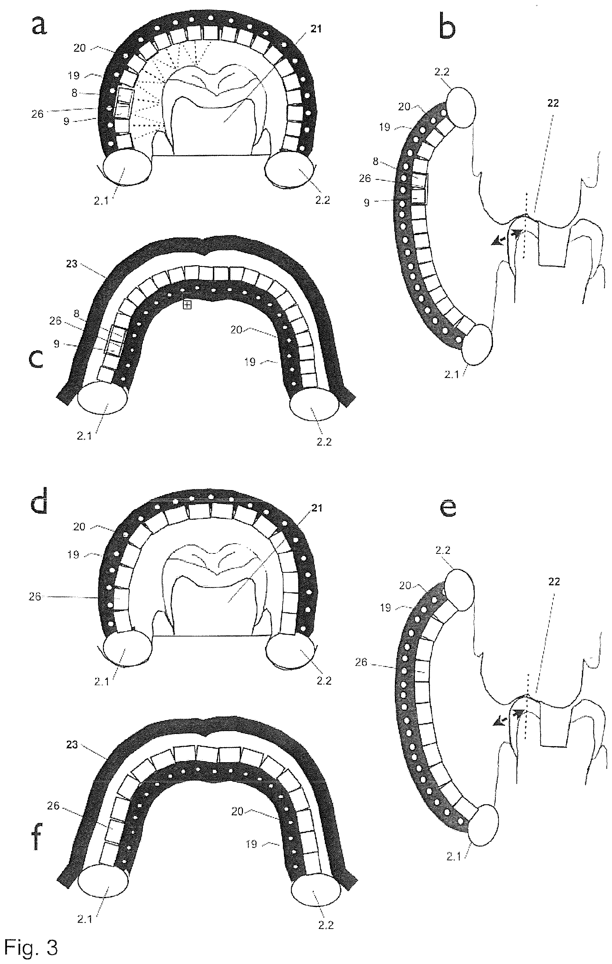

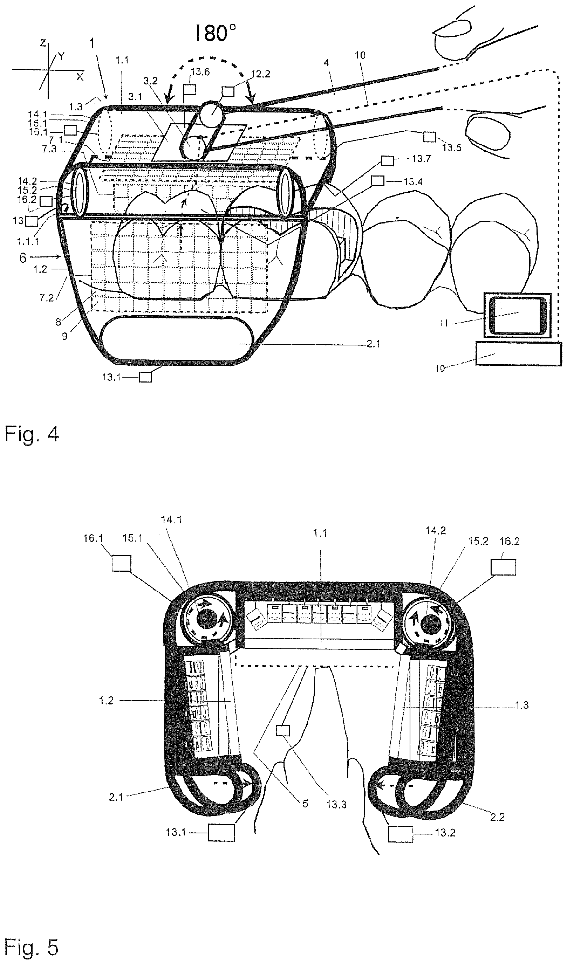

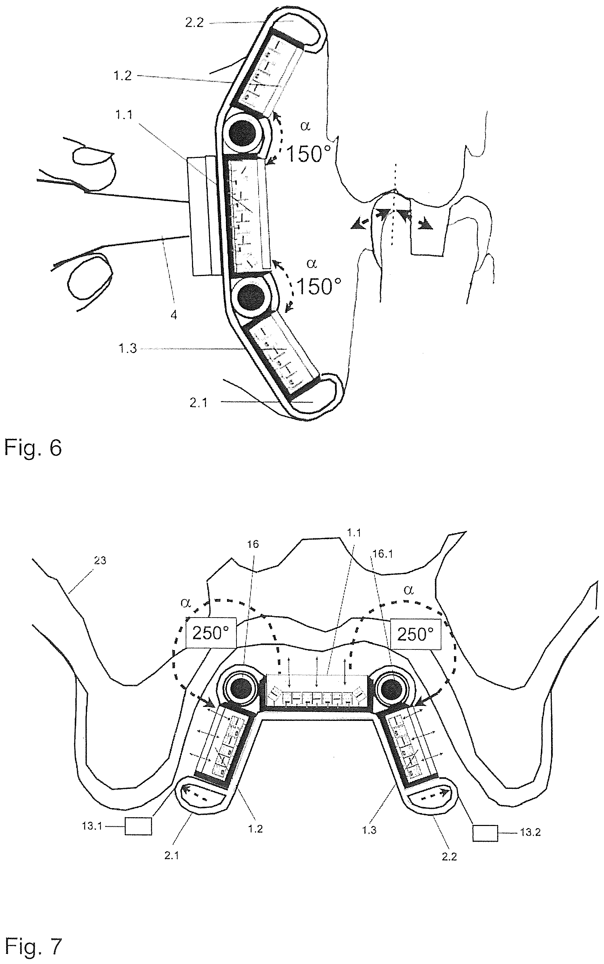

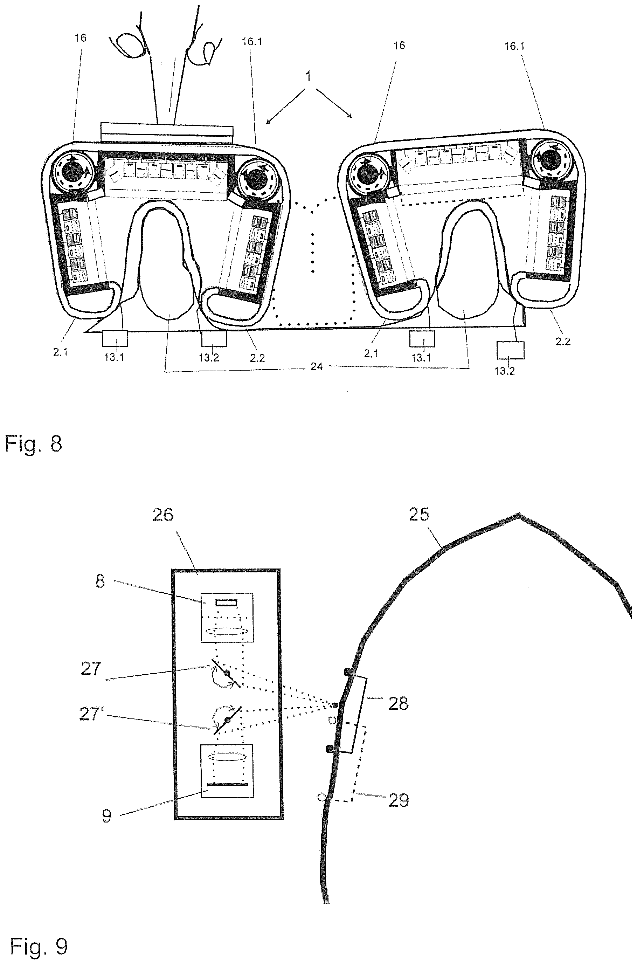

Die Verbindungsstellen zwischen den Messkörpern 1.1, 1.2 und 1.3, welche beispielsweise von den Scharnieren 14.1 und 14.2 gebildet werden, erlauben eine Veränderung der Winkelstellung der seitlichen Messkörper 1.2 und 1.3 gegenüber dem zentralen Messkörper 1.1 ausgehend von der in Fig.2 gezeigten senkrechten Stellung. Die Winkel können einzeln oder gemeinsam zumindest im Bereich von beispielsweise 1 - 30° nach innen (Fig.4), und 1 - 170° nach aussen verstellt werden, d.h. der messraumseitige Winkel α kann vorteilhaft zumindest im Bereich von 60° - 200° eingestellt werden. Winkeleinstellungen der seitlichen Messkörper 1.2 und 1.3 gegenüber dem zentralen Messkörper 1.1 zwischen α = 110° bis 180° nach aussen können mit besonderem Vorteil für die Vermessung des Schlussbisses von Ober- und Unterkiefer eingesetzt werden (Fig. 3, 6). Variationen der Winkelstellungen bis zu α = 270° der seitlichen Messkörper gegenüber dem zentaralen Messkörper eignen sich auch für die Vermessung zahnloser Unter- und Oberkiefer sowie des Gaumengewölbes (Fig. 7 und 8). Dies wird weiter unten genauer beschrieben.

Wie aus Fig. 2 anhand der Mikroprojektoren in Position 8i und 8ii illustriert, können in Bereichen des zentralen Messkörpers 1.1 und/oder der seitlichen Messkörper 1.2, 1.3 einzelne der Mikroprojektoren und/oder Mikrokameras gegenüber der jeweiligen Innenseite des Messkörpers unter einem Winkel ungleich 0 oder 90° angeordnet sein, um eine bessere Erfassung der Strukturen zu ermöglichen.

Alle auf der Innenseite mit Messeinheiten 26 besetzten Wandbereiche der Messkörper 1.1, 1.2, .1.3 können mit kratzfestem optischem Glas, beispielsweise Saphirglas, geschützt werden. So kann der Messraum im Wesentlichen aus diesem Material gebildet werden, welches dann bei Messkörper 1.1 auch die Funktion als Stütz-, Gleit- und Führungselement 1.1.1 erfüllen kann.

Um während der Führung des Messkopfes auf der Zahnreihe den Kontakt der Führungskörper 5, 2.1 und 2.2 mit der Zahnreihe zu sichern, können im Auflager 5 Kontakt- oder Distanzsensoren 13.3 untergebracht sein, wie auch und in den Führungskörpern 2.1 und 2.2 beidseits an den freien Enden der seitlichen Messkörper 1.2 und 1.3 (Fig. 2).

Die Übergänge zwischen den seitlichen Messkörpern 1.2 und 1.3 und dem zentralen Messkörper 1.1 im Bereich der Scharniere können vollständig mit einer Elastomerschicht 18, 18.1 abgedeckt sein, welche die Winkelveränderung der seitlichen Messkörper 1.2, 1.3 toleriert und den Messkopf allseitig verschliesst. Durch die geschlossene Konstruktion wird vorteilhaft eine effiziente Desinfektion des Messkopfes nach dem Einsatz am Patienten ermöglicht.

Fig. 3 zeigt schematische Schnittdarstellungen einer Messvorrichtung in verschiedenen Konfigurationen mit einem formbaren, flexibel anpassbaren Träger 19, der a) und d) über einem Seitenzahn 21, b) und e) auf der Bukkalseite im Seitenzahnbereich bei Schlussbiss und Artikulation 22 und c) und f) im Gaumengewölbe 23 der Topologie angepasst ist. In den Darstellungen a) bis c) sind Beispiele von Messeinheiten 26, mit Mikroprojektoren 8 und Mikrokameras 9 zu sehen, die jeweils entsprechend der Topologie der Zahnreihen ausgerichtet sind. Die Führungskörper 2.1 und 2.2 können als Abstützung der jeweiligen Konfiguration des Trägers 19 dienen. Die weissen punktförmigen Elemente 20 symbolisieren flexible Elemente beispielsweise Mikrogelenke oder deuten die Deformierbarkeit des Trägermaterials an.

In den Darstellungen gemäss Fig. 3 d) bis f) werden nochmals Ausführungen mit einzelnen Messeinheiten 26 gezeigt, die für beliebige Messprinzipien stehen können (z.B. Stereokamera-Paare für stereophotogrammetrische Verfahren, Baueinheiten für konfokale und interferometrische Verfahren etc). Dabei müssen nicht alle Messeinheiten 26 notwendigerweise identische Verfahren verwenden, sondern es können auf dem Träger auch Messeinheiten mit verschiedenen Messprinzipien kombiniert werden.

Fig. 4 zeigt eine schematische Skizze einer weiteren Ausführung der Vorrichtung. Der Messkopf 1 dieser Konfiguration ist um die Achse Y beweglich mit einem Schaft 4 verbunden. Der Schaft 4 kann beispielsweise zwischen 40 - 150 mm lang sein, eine Gesamthöhe (in Richtung der Achse Z) von 5 - 30 mm aufweisen und in Richtung der Achse Y beispielsweise zwischen 18 - 36 mm breit sein.

Die Steuerzuleitungen 10 zu den einzelnen Sensorarrays 7.1, 7.2, 7.3 werden durch den Messkopf 1 geführt und gelangen durch den Schaft 4 zu einem Steuergerät 10, wo auf einem Monitor 11 die zusammengeführten Einzelaufnahmen der Messeinheiten 26 als Gesamtaufnahme der Zahnreihe dargestellt werden können.

Die Messkörper 1.1, 1.2 und 1.3 können wiederum mit Mikroprojektoren-Mikrokamera-Arrays 7.1, 7.2, 7.3 mit beispielsweise je 36 Mikroprojektoren 8 und 36 Mikrokameras 9 besetzt sein, die mit einem Querschnittsmass von beispielsweise 1x1 mm alternierend angeordnet sein können, wobei sich die Messeinheiten 26 in Form von Arrays kontinuierlich oder in separaten Einheiten aneinander fügen können.

Die Vielzahl der optischen Messeinheiten 26 (jede einzelne z.B. bestehend aus einem Mikroprojektor 8 und einer Mikrokamera 9) erlaubt es, diese innerhalb von Arrays vorteilhaft zeilen- oder spaltenweise auf unterschiedliche Raumwinkel auszurichten, oder zwischen unterteilten Arrays verschiedene Ausrichtungen der Raumwinkel anzuwenden.

Alle Messeinheiten 26, oder auch nur ein Teil der Messeinheiten (z.B. ein Teil der Mikroprojektoren 8 und Mikrokameras 9), können für die Darstellung der Zahnreihen und der Mundschleimhaut in Echtfarben eingesetzt werden. Damit wird eine möglichst naturähnliche ästhetische Gestaltung der Zahnrestaurationen ermöglicht.

Die Beweglichkeit des Schaftes 4 gegenüber dem Messkopf 1 um die Achse Y um 180° erlaubt es zudem, den Messkopf 1 gegenüber der Zahnreihe um 180° umgekehrt aufzusetzen. Der Messkopf 1 kann somit vorteilhaft in zwei Läufen vom linken in den rechten Mundbereich und vom rechten in den linken Mundbereich geführt werden oder umgekehrt. Dabei wird der Schaft 4 beim Durchgang durch die Kiefermitte auf die jeweils andere Seite geschlagen.

Falls eine oder mehrere Messeinheiten in den Messkörpern 1.1, 1.2, 1.3 in Richtung der Achse X mit einem Blickwinkel von 2°- 45° angestellt sind, und die Zahnreihe mit einem solchen generellen Blickwinkel in dieser Richtung vermessen wird, kann die Vermessung durch einen zweiten Messdurchgang mit umgekehrtem Messkopf 1 und damit entgegengesetztem Blickwinkel ergänzt werden. So können vorteilhaft zusätzliche Bilddaten erzeugt werden. Die Winkelstellungen des Führungsschaftes 4 können durch einen Winkelsensor 12.2 erfasst werden. Der beim Scannen auf den Messkopf ausgeübte Druck wird durch einen Drucksensor 13.6 in der Führungsbasis 3.1 erfasst. Winkel- und Druckdaten können zur Steuerung des Messkopfes mit Hilfe eines Mikromotors beispielsweise mit 4 mm Durchmesser im Verbindungsteil 3.2 verwendet werden.

Zur Erweiterung des Messraums und zur Anpassung an unterschiedliche anatomische Kiefer-Topologien kann die Winkelstellung beider seitlicher Messkörper 1.2 und 1.3 gegenüber dem kauflächenseitigen Messkörper 1.1 mittels der Scharniere 14.1 und 14.2 durch eingebaute Mikromotoren 15.1, 15.2 mit einem Durchmesser von beispielsweise 4 mm verstellt und durch Winkelsensoren 16.1 bzw. 16.2 kontrolliert werden.

Um während der Führung des Messkopfes auf der Zahnreihe den Kontakt der Führungskörper 1.1.1, 2.1 und 2.2 mit der Zahnreihe zu ermöglichen, sind in den das Führungselement 1.1.1 umgebenden distalen und mesialen Wandteilen Kontakt- oder Distanzsensoren 13 und 13.1 und in den seitlichen Rahmenteilen je die Kontakt- oder Distanzsensoren 13.2 und 13.3 untergebracht. Weitere Kontakt- oder Distanzsensoren 13.4, 13.5 befinden sich in den Führungskörpern 2.1 und 2.2 beidseits an den freien Enden der seitlichen Messkörper 1.2 und 1.3 (Fig. 4).

### Messeinheiten

Die Messeinheiten 26 können, wie erwähnt, mindestens teilweise von Mikroprojektoren und Kameras gebildet werden, welche nach dem Prinzip des strukturierten Lichts arbeiten, wie es z.B. in EP 2166303 oder EP 2469224 beschrieben ist.

Die miniaturisierten Mikroprojektoren bestehen jeweils einzeln oder in einer Systemanordnung je aus Lichtquellen, die eine farbige LED, eine weisse LED oder ein Laser sein können oder die aus Lichtleitern gespeist werden können. Für das Triangulationsverfahren kann sowohl polychromatisches als auch monochromatisches Licht eingesetzt werden. Die Lichtquellen der Mikroprojektoren zur Projektion einzelner Muster können sich in der Wellenlänge unterscheiden, damit die Muster in gleichzeitigen Triangulationsaufnahmen aus verschiedenen Raumwinkeln unabhängig voneinander ausgewertet werden können.

Die Mikroprojektoren können mit unterschiedlichen Masken zur Erzeugung von unterschiedlichen Mustern, beispielsweise Streifenmustern mit unterschiedlicher Erstreckungsrichtung der Streifen, bestückt sein. Alternativ können sie für die Projektion der Streifenmuster als Bildmatrix-Projektor ausgebildet sein, der durch ein Videosignal gesteuert wird. Vorteilhaft können die Mikroprojektor-Elemente aus einem miniaturisierten digitalen DLP Mustergenerator (Texas Instruments Application Report DLPA026-May 2011) oder LCoS- Musterprojektor bestehen und können eine Mikroprojektor-Optik mit einer oder mehreren Linsen aufweisen.

Vorzugsweise bestehen die Mikrokamera-Elemente aus einem CCD-Sensor oder einem CMOS-Sensor und einer Mikrokameraoptik aus einer oder mehreren Linsen. Beispielsweise können 'NanEye CSP' (www.awaiba.com) Kameramodule verwendet werden, die einen 1 mm x 1 mm grossen CMOS Bildsensor aufweisen und mit einer angepassten Miniaturoptik ausgestattet werden können. Vorzugsweise sind diese Elemente in einem Array integriert.

Der von den Messeinheiten 26 erzeugte Strahlengang kann mithilfe schwenkbarer Spiegel in unterschiedliche Messrichtungen gebracht werden. Hierzu können die Messeinheiten 26 in Form von Mikrospiegelarrays angeordnet sein (Ref. 9).

Ein Vorteil des beschriebenen Verfahrens der optischen dreidimensionalen Vermessung ist, dass einerseits ein einzelnes, auf die Oberfläche der Zahnreihe durch einen Mikroprojektor projiziertes Muster von mehreren benachbarten Mikrokameras aus unterschiedlichen Raumwinkeln gleichzeitig aufgenommen und die Bilddaten unabhängig voneinander ausgewertet werden können. Andererseits können von den eine Mikrokamera unmittelbar umgebenden, beispielsweise vier Mikroprojektoren gleichzeitig Farbstreifenmuster in unterschiedlichen Spektralbereichen aus unterschiedlichen Raumwinkeln projiziert werden, die von einer zugeordneten Mikrokamera gleichzeitig aufgenommen und die unabhängig voneinander per Binärkode sofort ausgewertet werden können. Hierzu können z.B. durch die Verwendung von Filtern spektral unterscheidbare Farbmuster erzeugt werden. In beiden Fällen kann eine Vielzahl von Bilddaten aus unterschiedlichen Raumwinkeln gewonnen werden.

Vorteilhaft können die von den zentralen und seitlichen Messeinheiten/Arrays ausgehenden Messungen gleichzeitig oder sequentiell erfolgen.

Auf den einzelnen Arrays können die von den einzelnen Messeinheiten ausgehenden Messungen in den Zeilen und Spalten gleichzeitig oder sequentiell erfolgen.

Mikroprojektoren und Mikrokameras können selektiv mit Optiken mit unterschiedlichen Schärfentiefen ausgestattet sein, wodurch der Schärfentiefenbereich erweitert und die höchste effektive Auflösung der dreidimensionalen Vermessung der Zahnreihe erzielt werden kann.

Typisch weisen die Miniaturoptiken der Mikroprojektor-Mikrokamera-Elemente einen Schärfentiefenbereich von 1 - 20 mm auf.

Besonders vorteilhaft können zur Erweiterung des Schärfentiefenbereichs fokusadaptive Linsen für die optische Ausstattung der Messeinheiten eingesetzt werden.

Anstelle (oder zusätzlich zu) einer Messung mittels strukturiertem Licht kann die Messung mittels stereoskopischen Kameras erfolgen. Es kann auch eine Kombination von Triangulationsverfahren und stereoskopischen Verfahren eingesetzt werden. Weiter kann auch die konfokale Lasertechnologie zur Messung eingesetzt werden.

### Messverfahren, Anwendungen

Fig. 5 illustriert eine spezielle Anwendung der Vorrichtung gemäss Fig.1 bei der Vermessung eines zentralen Frontzahnes mit deutlich schmalerem Alveolarfortsatz als im Seitenzahnbereich (Fig.2). In der Darstellung ist beispielsweise der Messkörper 1.2 um 6° und der Messkörper 1.3 um 17° nach innen geschwenkt.

Fig. 6 illustriert eine spezielle Anwendung der Vorrichtung bei der Vermessung des Schlussbisses beider Kiefer. Dargestellt ist die Vorrichtung gemäss Fig. 4, wobei der Träger (d.h. die Messkörper 1.1, 1.2, 1.3) sich jedoch in einer anderen Konfiguration befindet.

Der Messkopf 1 kann durch gleichzeitige Abstützung der Stützelemente 2.1 und 2.2 auf der Mundschleimhaut der Alveolarfortsätze des Ober- und Unterkiefers mit dem Schaft 4 abgestützt werden.

Die Aufnahmeaktivität kann auf das zentrale Array 1.1 im Messkörper 1.1 beschränkt werden.

Bei Verwendung der Vorrichtung gemäss Fig.1 kann die Vermessung sowohl mit als auch ohne Aktivierung des Kippmodus des Messkopfes erfolgen. Die Abstützung kann auf jeden Fall auf dem Auflager 5 am Schaft 4 auf den Bukkalflächen der Zahnreihe erfolgen.

Aus der dargestellten Position können mit hoher Bildfolge ca. 18 - 24 Aufnahmen pro Sekunde oder mehr auch die Artikulationsbewegungen der Unterkiefers gegen den Oberkiefer aufgenommen werden.

Fig. 7 zeigt die schematische Skizze eines Querschnitts durch den zahnlosen Oberkiefer 23 bei einer weiteren Anwendung des erfindungsgemässen Vermessungsverfahrens.

Hier ist der Träger (gebildet durch die Messkörper 1.1, 1.2, 1.3) in einer konvexen Konfiguration. Die seitlichen Messkörper 1.2 und 1.3 des Messkopfes werden bei dieser Anwendung entsprechend der Wölbung des Gaumens um einen Winkel α von beispielsweise 250° über die Ebene des zentralen Messkörpers 1.1 hinaus geschwenkt, bis die Messkörper annähernd parallel zur Gaumenoberfläche stehen. Der Messkopf kann bei Einsatz der Ausführung gemäss Fig. 1 (mit Kippaktivierung) unter Abstützung auf dem Auflager 5 des Schaftes 4 über den Gaumen geführt werden.

Bei Einsatz der Vorrichtung gemäss Fig. 4 kann der Messkopf mit mindestens einem der Führungskörper 2.1 oder 2.2 in Kontakt mit der Mundschleimhaut gebracht und translatorisch sowie rotierend am Gaumen bewegt werden, bis die gesamte Oberfläche vermessen ist. Zur optimalen Abstützung können die seitlichen Messkörper 1.2 und 1.3 mit ihren Führungskörpern 2.1 und 2.2 durch die Kontakt- oder Distanzsensoren 13.1 und 13.2 über die Steuerung der Mikromotoren in den Scharnieren während der Scanbewegungen entlang der Gaumenoberfläche in vollständigem Kontakt gehalten werden. Dadurch können vorteilhaft annähernd gleichmässige Abstände für die dreidimensionale optische Vermessung geschaffen werden.

Die Vermessung der gesamten Gaumenoberfläche ist sowohl für prothetische als auch für kieferorthopädische Behandlungen wesentlich.

Fig. 8 zeigt die schematische Skizze eines Querschnitts durch den zahnlosen Unterkiefer 24 bei einer weiteren Anwendung der optisch dreidimensionalen geführten Vermessung. Die Vermessung kann mit dem Messkopf 1 sowohl mit dem Schaft 4 gemäss Fig. 1 als auch mit dem Schaft 4 gemäss Fig. 4 ausgeführt werden. Zur optimalen Abstützung können die seitlichen Messkörper 1.2 und 1.3 mit ihren Führungskörpern 2.1 und 2.2 durch die Kontakt- oder Distanzsensoren 13.1 und 13.2 über die Steuerung der Mikromotoren in den Scharnieren während der Scanbewegungen entlang der Oberfläche des gesamten Unterkieferkammes die Führung der Messvorrichtung gewährleisten.

Fig. 9 zeigt eine schematische Skizze einer Messeinheit 26 bestehend aus einem Mikroprojektor 8 und einer Mikrokamera 9 mit Mikrospiegeln 27, 27' und einem ersten Messbereich 28 und einem zweiten Messbereich 29 auf einer Zahnoberfläche 25.

Diese Ausführung illustriert, dass die Anordnung des Messbereichs einer Messeinheit auch dann variiert werden kann, wenn sich der Träger des Messkopfs nicht bewegt.

Die Ausführung nach Fig. 9 kann jedoch auch mit einem beweglichen, d.h. konfigurierbaren, Träger kombiniert werden.

### Bemerkungen

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

### Referenzen

1. Vollborn T, Habor D, Pekam FC, Heger S, Marotti J, Reich S, Wolfart S, Tinschert J, Radermacher K.: Soft tissue-preserving computer-aided impression: a novel concept using ultrasonic 3D-scanning. Int J Comput Dent. 2014; 277-96.
2. Yueli L. Chen, Quan Zhang and Quing Zhu (2012). Optical Coherence Tomography in Dentistry, Selected Topics in Optical Coherence Tomography, Dr. Gangjun Liu (Ed.), ISBN: 978-953-51-0034-8, InTech, Available from: http://www.intechopen.com/books/selected-topics-in-optical-coherence-tomography/optical-coherence-tomography-in-dentistry
3. Yao-Sheng Hsieh, Yi-Ching Ho, Shyh-Yuan Lee, Ching-Cheng Chuang, Jui-che Tsai, Kun-Feng Lin and Chia-Wei Sun: Dental Optical Coherence Tomography. Sensors 2013, 13, 8928-8949; doi:10.3390/s130708928.
4. Malchow, Doug: Optical Coherence Tomography/Dentistry: Driving OCT into Dentistry. BioOptics World 7/1 (2014).
5. Shankar Krishnan, Pei Yean Lee, John B Moore, Suresh Venkatasubramanian: Optimization-on-a-manifold for global registration of multiple 3D point sets. Eurographics Symposium on Geometry Processing M. Editors: Desbrun, H. Pottmann, 2005, 1-11.
6. Fisher B, McDonagh S. Simultaneous registration of multi-view range images with adaptive kernel density estimation. In The Mathematics of Surfaces - XIV. Institute of Mathematics and its Applications. 2013. p. 31-62.
7. Neugebauer J, Kistler S, Elbertzhagen A, Kistler F. Digitale Hilfsmittel zur Optimierung prothetischer Versorgungen. BZB Juli/August 2013: 72-76. http://www.cumdente.com/fileadmin/user_upload/doc/BZB_7-8-2013_Dr.Neugebauer_T-Scan.pdf
8. Koos B, Godt A, Schille C, Göz G.: Precision of an instrumentation-based method of analyzing occlusion and its resulting distribution of forces in the dental arch. J Orofac Orthop. 2010 Nov;71 (6):403-10. doi: 10.1007/s00056-010-1023-7. Epub 2010 Nov 17.
9. Sonderforschungsbereich 379, Mikromechanische Sensor- und Aktorarrays, Scanner http://www.zfm.tu-chemnitz.de/pdf/sfb379_finalslides/scanner.pdf; Folie 20.

## Patentansprüche

1. Messvorrichtung zum Ausmessen mindestens eines Teils eines Mundraums, insbesondere mindestens eines Teils einer Zahnreihe, mit einem Messkopf (1), der Messkopf (1) aufweisend
mindestens zwei Messeinheiten (26), wobei jede Messeinheit (26) einen Messbereich besitzt und wobei die Messbereiche so anordenbar sind, dass sie unterschiedliche Areale des Teils einer Zahnreihe erfassen, und
einen Träger (1.1, 1.2, 1.3; 19), welcher die Messeinheiten (26) trägt,
**dadurch gekennzeichnet, dass** jede Messeinheit (26) dazu in der Lage ist, zumindest eine Oberfläche in ihrem Messbereich dreidimensional zu erfassen, wobei eine relative Anordnung der Messbereiche mindestens zweier der Messeinheiten (26) verstellbar ist.

2. Messvorrichtung nach Anspruch 1, wobei der Träger mindestens in eine erste und eine zweite Konfiguration bringbar ist, wobei die relative Anordnung der Messbereiche mindestens zweier der Messeinheiten (26) sich in der ersten und der zweiten Konfiguration unterscheidet.

3. Messvorrichtung nach Anspruch 2, wobei der Träger (1.1, 1.2, 1.3; 19) mindestens teilweise elastisch oder plastisch deformierbar ist, um ihn reversibel von der ersten in die zweite Konfiguration und von der zweiten in die erste Konfiguration überzuführen.

4. Messvorrichtung nach einem der Ansprüche 2 oder 3, wobei der Träger (1.1, 1.2, 1.3; 19) mindestens zwei zueinander bewegliche Messkörper (1.1, 1.2, 1.3) aufweist, wobei an jedem Messkörper (1.1, 1.2, 1.3) mindestens eine Messeinheit (26) angeordnet ist.

5. Messvorrichtung nach einem der vorangehenden Ansprüche, wobei der Messkopf (1) einen Messraum (6) definiert, der in mindestens einer Konfiguration des Trägers entlang einer Achse X beidseitig offen ist, entlang einer Achse Y beidseitig vom Messkopf (1) begrenzt ist und entlang einer Achse Z einseitig offen und einseitig vom Messkopf (1) begrenzt ist, wobei die Achsen X, Y und Z senkrecht zueinander angeordnet sind

6. Messvorrichtung nach Anspruch 5, wobei der Messkopf (1) aufweist:
mindestens zwei seitliche Führungskörper (2.1, 2.2) mit Führungsbereichen zum seitlichen Anlegen an die Zahnreihe und
mindestens zwei seitliche Messkörper (1.2, 1.3), wobei wenigstens ein Teil der Messeinheiten (26) in den seitlichen Messkörpern (1.2, 1.3) angeordnet ist,
und insbesondere wobei der Messkopf (1) so konfiguriert oder konfigurierbar ist, dass entlang der Achse Z der Abstand (Dz) der Führungsbereiche der Führungskörper (2.1, 2.2) von einem den Messbereich entlang Achse Z begrenzenden Teil (1.1.1) des Messkörpers (1) zwischen 5 und 20 mm liegt und entlang der Achse Y der Abstand (Dy) der Führungsbereiche der seitlichen Führungskörper (2.1, 2.2) voneinander zwischen 10 und 24 mm, insbesondere 14 und 22 mm, liegt.

7. Messvorrichtung nach Anspruch 6, wobei die seitlichen Messkörper (1.2, 1.3) und/oder die seitlichen Führungskörper (2.1, 2.2) zueinander verschwenkbar angeordnet sind, und insbesondere wobei die seitlichen Messkörper (1.2, 1.3) und/oder die seitlichen Führungskörper (2.1, 2.2) zueinander um mindestens 30°, insbesondere mindestens 90°, schwenkbar sind.

8. Messvorrichtung nach Anspruch 7 mit mindestens einem Stellungssensor (16.1, 16.2), mit welchen eine Schwenkpositionen der seitlichen Messkörper (1.2, 1.3) und/oder die seitlichen Führungskörper (22.2, 22.3) erfassbar ist.

9. Messvorrichtung nach einem der Ansprüche 6 bis 8, wobei der Messkopf (1) weiter einen zentralen Messkörper (1.1) aufweist, an welchem die seitlichen Messkörper (1.2, 1.3) schwenkbar angeordnet sind,
und insbesondere wobei
- ein Abstand der seitlichen Führungskörper (2.1, 2.2) um mindestens 3 mm variierbar ist und/oder
- die seitlichen Messkörper (1.2, 1.3) quer, insbesondere senkrecht, zum zentralen Messkörper (1.1) anordenbar sind.

10. Messvorrichtung nach Anspruch 9, wobei auf einer dem Messraum (6) zugewandten Seite des zentralen Messkörpers (1.1) ein Stützelement (1.1.1) angeordnet ist, welches im Bereich der Messeinheiten (26) transparent oder ausgespart ist.

11. Messvorrichtung nach einem der Ansprüche 6 bis 10 mit einem Antrieb (15.1, 15.2), um die seitlichen Messkörper (1.2, 1.3) gegeneinander zu verschwenken,
und insbesondere wobei die Messvorrichtung Sensoren (13.1 - 13.14, 16.1, 16.2) zum Steuern des Antriebs (15.1, 15.2) aufweist und/oder wobei mit dem Antrieb (15.1, 15.2) die seitlichen Messkörper (1.2, 1.3) in vordefinierte Positionen schwenkbar sind.

12. Messvorrichtung nach einem der Ansprüche 6 bis 11, wobei die seitlichen Messkörper (1.2, 1.3) sich entlang der Achse X über eine Länge von 3 bis 12 mm, insbesondere 4 bis 8 mm, erstrecken, und/oder wobei sich die seitlichen Führungskörper (22.2, 22.3) entlang der Achse X über eine Länge von 4 bis 30 mm, insbesondere 6 bis 16 mm, erstrecken.

13. Messvorrichtung nach einem der Ansprüche 6 bis 12, wobei die seitlichen Führungskörper (2.1, 2.2) an den seitlichen Messkörpern (1.2, 1.3) angeordnet sind, insbesondere entlang von dem zentralen Messkörper (1.1) gegenüber liegenden Kanten der seitlichen Messkörper (1.2, 1.3).

14. Messvorrichtung nach einem der vorangehenden Ansprüche, wobei am Messkopf (1) ein Schaft (4) mit einer Länge von mindestens 5 cm, insbesondere mindestens 10 cm, angeordnet ist, und insbesondere wobei zwischen dem Schaft (4) und dem Messkopf (1) eine bewegliche Verbindung (12) angeordnet ist, und insbesondere wobei der Schaft (4) gegenüber dem Messkopf (1) um die Achse Z um mindestens 60° drehbar ist.

15. Messvorrichtung nach einem der vorangehenden Ansprüche mit
einem Auflager (5) zur Abstützung des Messkopfs (1) auf einer Zahnreihe und
einem Kippaktuator (3), mit welchem der Träger (1.1, 1.2, 1.3) gegenüber dem Auflager (5) automatisiert verkippbar ist.

16. Messvorrichtung nach den Ansprüchen 5 und 15, wobei mit dem Kippaktuator der Träger (1.1, 1.2, 1.3) gegenüber dem Auflager (5) um die Achse Y und/oder die Achse Z verkippbar ist.

17. Messvorrichtung nach einem der vorangehenden Ansprüche, wobei die Messeinheiten (26) Mikroprojektoren (8) und Mikrokameras (9) aufweisen, und insbesondere wobei die Mikroprojektoren (8) und Mikrokameras (9) Teile des Messkopfs (1) bilden und/oder wobei die Mikroprojektoren (8) und Mikrokameras (9) in Arrays sowohl zeilenweise als auch spaltenweise alternierend angeordnet sind, insbesondere wobei die Mikroprojektoren (8) ein Muster aus überkreuzten Streifen erzeugen.

18. Messvorrichtung nach den Ansprüchen 5 und 17, wobei die Mikroprojektoren (8) und/oder Mikrokameras (9) gegenüber der Achse X unterschiedliche Winkelstellungen, insbesondere in einem Bereich von 2 - 45°, einnehmen, und/oder wobei Piezo-Aktoren zum einzelnen oder gruppenweisen Auslenken der Mikroprojektoren (8) und/oder Mikrokameras (9) vorgesehen sind.

19. Messvorrichtung nach einem der vorangehenden Ansprüche mit mindestens einem Kontakt- oder Distanzsensor (13.1, 13.2), um einen Kontakt des Messkopfs (1) mit dem auszumessenden Mundraum zu detektieren, und insbesondere wobei die Messvorrichtung derart ausgestaltet ist, dass sie eine Konfiguration des Trägers abhängig vom Signal des Kontakt- oder Distanzsensors (13.1, 13.2) variiert.

20. Messvorrichtung nach einem der vorangehenden Ansprüche, zum Ausmessen mindestens eines Teils einer Zahnreihe, wobei der Messkopf (1) einen Messraum definiert, der entlang einer Achse X beidseitig offen ist, entlang einer Achse Y beidseitig vom Messkopf (1) begrenzt ist und entlang einer Achse Z einseitig offen und einseitig vom Messkopf (1) begrenzt ist, wobei die Achsen X, Y und Z senkrecht zueinander angeordnet sind und
wobei der Messkopf (1) aufweist:
- mindestens zwei seitliche Führungskörper (2.1, 2.2) mit Führungsbereichen zum seitlichen Anlegen an die Zahnreihe und
- mindestens zwei seitliche Messkörper (1.2, 1.3), wobei wenigstens ein Teil der Messeinheiten (26) in den seitlichen Messkörpern (1.2, 1.3) angeordnet ist,
und wobei der Messkopf (1) so konfiguriert oder konfigurierbar ist, dass entlang der Achse Z der Abstand (Dz) der Führungsbereiche von einem den Messbereich entlang Achse Z begrenzenden Teil des Messkörpers (1) zwischen 5 und 20 mm liegt und entlang der Achse Y der Abstand (Dy) der Führungsbereiche der seitlichen Führungskörper (22.2, 22.3) voneinander zwischen 10 und 24 mm, insbesondere 14 und 22 mm, liegt.

21. Verfahren zum Ausmessen mindestens eines Teils eines Mundraums mit einer Vorrichtung nach einem der vorangehenden Ansprüche umfassend die Schritte
a) Positionieren des Messkopfs (1) über einer Struktur des Mundraums, insbesondere über einer Zahnreihe, derart dass die Struktur in einen Messraum (6) des Messkopfs zu liegen kommt, und insbesondere wobei die Messkörper (1.1, 1.2, 1.3) mindestens mit ihren seitlichen Führungskörpern (2.1, 2.2) mit der Struktur in Berührung kommen,
b) Führen des Messkopfs entlang der Struktur, insbesondere derart, dass mindestens ein seitlicher Führungskörper mit der Struktur in Berührung bleibt, und
c) Ausmessen der Struktur, insbesondere während dem Führen des Messkopfs.

22. Verfahren nach Anspruch 21, wobei die Messvorrichtung einen Schaft (4) und einen elektronisch gesteuerten Kippaktuator (3) aufweist, um den Messkopf (1) um mindestens eine Achse gegenüber dem Schaft (4) zu kippen,
wobei während dem Ausmessen der Struktur der Messkopf gegenüber dem Schaft (4) der Messvorrichtung mit dem Kippaktuator in unterschiedliche Stellungen gekippt wird, um Messaufnahmen aus unterschiedlichen Blickwinkelpositionen zu erzeugen

23. Verfahren nach einem der Ansprüche 21 oder 22, wobei die Konfiguration des Trägers beim Führen des Messkopfs (1) entlang der Struktur variiert wird.

24. Verfahren nach einem der Ansprüche 21 bis 23, wobei der Messkopf (1) sowie ein Schaft (4) der Messvorrichtung kauebenenseitige Führungskörper (1.1.1; 5) zum Anlegen an einer Zahnreihe aufweist und wobei beim Ausmessen der Zahnreihe kauebenenseitige Führungskörper (1.1.1; 5) kauebenenseitig an der Zahnreihe anliegen.

25. Verfahren nach Anspruch 24, wobei der Messkopf (1) seitliche Führungskörper (2.1, 2.2) aufweist und die seitlichen Führungskörper (2.1, 2.2) beim Ausmessen in Anschlag mit Seitenflächen von Zähnen der Zahnreihe gebracht werden, oder wobei die seitlichen Führungskörper (2.1, 2.2) beim Ausmessen in Anschlag mit der Mundschleimhaut der Zahnreihe gebracht werden.

26. Verfahren nach einem der Ansprüche 21 bis 25, wobei zur Ausmessung der Messkopf (1) ohne Absetzen entlang mindestens einer halben Zahnreihe, insbesondere einer ganzen Zahnreihe, geführt wird.

27. Verfahren nach Anspruch 26, wobei der Messkopf (1) in zwei Läufen von einem linken in einen rechten Mundbereich und vom rechten in den linken Mundbereich geführt wird, oder umgekehrt, wobei der Messkopf (1), zwischen den Läufen abgenommen, um 180° gedreht und wieder aufgesetzt wird.

28. Verfahren nach einem der Ansprüche 21 bis 27, wobei das Führen des Messkopfs (1) kontinuierlich erfolgt, und insbesondere wobei die Messvorrichtung (1) über Ausgabemittel angibt, ob eine Führgeschwindigkeit korrekt ist,
oder wobei das Verschieben des Messkopfs (1) intervallweise erfolgt, insbesondere wobei die Messvorrichtung über Ausgabemittel angibt, wann der Messkopf (1) weiterzuführen ist.

## Claims

1. Measuring apparatus for measuring at least a part of an oral cavity, particularly at least a part of a row of teeth, with a measuring head (1), the measuring head (1) comprising
at least two measuring units (26), wherein each measuring unit (26) has a measuring zone and wherein the measuring zones are arranged such that they acquire different areas of the part of a row of teeth, and
a carrier (1.1, 1.2, 1.3; 19) carrying the measuring units (26),
**characterized in that** each measuring unit (26) is capable of acquiring three-dimensionally at least a surface in its measurement range, wherein a relative arrangement of the measurement ranges of at least two of the measuring units (26) are displaceable.

2. Measuring apparatus according to claim 1 wherein the carrier is brought at least in a first and a second configuration, wherein the relative arrangement of the measuring zones of at least two of the measuring units (26) differs in the first and the second configuration.

3. Measuring apparatus according to claim 2 wherein the carrier (1.1, 1.2, 1.3; 19) is at least in part elastically or plastically deformable for converting it reversibly from the first into the second configuration and from the second into the first configuration.

4. Measuring apparatus according to one of the claims 2 or 3, wherein the carrier (1.1, 1.2, 1.3; 19) comprises at least two measuring members (1.1, 1.2, 1.3) which are movable with respect to each other, wherein at least one measuring unit (26) is arranged at each measuring member (1.1, 1.2, 1.3).

5. Measuring apparatus according to one of the preceding claims, wherein the measuring head (1) defines a measuring space (6), which in at least one configuration of the carrier is open on both sides along an axis X, is confined along an axis Y on both sides by the measuring head (1), and is open on one side and confined on one side by the measuring head (1) along an axis Z, wherein the axes X, Y and Z are arranged orthogonally with respect to each other.

6. Measuring apparatus according to claim 5 wherein the measuring head (1) comprises:
at least two lateral guiding members (2.1, 2.2) with guiding zones for the lateral abutting on the row of teeth and
at least two lateral measuring members (1.2, 1.3), wherein at least a part of the measuring units (26) is arranged in the lateral measuring members (1.2, 1.3),
and particularly wherein the measuring head (1) is configured or configurable such that along the axis Z the distance (Dz) of the guiding zones of the guiding members (2.1, 2.2) from a part (1.1.1) of the measuring member (1) confining the measuring space along the axis Z is between 5 and 20 mm and along the axis Y the distance (Dy) of the guiding zones of the lateral guiding members (2.1, 2.2) from each other is between 10 and 24 mm, particularly between 14 and 22 mm.

7. Measuring apparatus according to claim 6, wherein the lateral measuring members (1.2, 1.3) and/or the lateral guiding members (2.1, 2.2) are arranged in a pivotable way with respect to each other, and particularly wherein the lateral measuring members (1.2, 1.3) and/or the lateral guiding members (2.1, 2.2) are arranged in a pivotable way with respect to each other by at least 30°, particularly at least 90°.

8. Measuring apparatus according to claim 7 with at least one position sensor (16.1, 16.2) by means of which a pivoting position of the lateral measuring members (1.2, 1.3) and/or of the lateral guiding members (22.2, 22.3) is measurable.

9. Measuring apparatus according to one of the claims 6 to 8, wherein the measuring head (1) further comprises a central measuring member (1.1) at which the lateral measuring members (1.2, 1.3) are arranged in a pivotable way,
and particularly wherein
- a distance of the lateral guiding members (2.1, 2.2) is variable by at least 3 mm and/or
- the lateral measuring members (1.2, 1.3) are arranged transversally, particularly perpendicularly to the central measuring member (1.1).

10. Measuring apparatus according to claim 9 wherein a supporting element (1.1.1) is arranged on a side facing the measuring space (6) of the central measuring member (1.1), which is transparent or recessed in the area of the measuring units (26).

11. Measuring apparatus according to one of the claims 6 to 10, with an actuator (15.1, 15.2) for pivoting the lateral measuring members (1.2, 1.3) with respect to each other,
and particularly wherein the measuring apparatus comprises sensors (13.1 - 13.14, 16.1, 16.2) for controlling the actuator (15.1, 15.2) and/or wherein by means of the actuator (15.1, 15.2) the lateral measuring members (1.2, 1.3) can be pivoted into predefined positions.

12. Measuring apparatus according to one of the claims 6 to 11, wherein the lateral measuring members (1.2, 1.3) extend along the axis X over a length of 3 to 12 mm, particularly 4 to 8 mm and/or wherein the lateral guiding members (22.2, 22.3) extend along the axis X over a length of 4 to 30 mm, particularly 6 to 16 mm.

13. Measuring apparatus according to one of the claims 6 to 12, wherein the lateral guiding members (2.1, 2.2) are arranged at the lateral measuring members (1.2, 1.3), particularly along the edges of the lateral measuring members (1.2, 1.3) opposing the central measuring member (1.1).

14. Measuring apparatus according to one of the preceding claims, wherein a shaft (4) is arranged at the measuring head (1), with a length of at least 5 cm, particularly at least 10 cm, and particularly wherein a movable connection (12) is arranged between the shaft (4) and the measuring head (1), and particularly wherein the shaft (4) is rotatable around the axis Z with respect to the measuring head (1) by at least 60°.

15. Measuring apparatus according to one of the preceding claims, with
a support (5) for stabilizing the measuring head (1) on a row of teeth and
a tilting actuator (3) with which the carrier (1.1, 1.2, 1.3) is automatically tiltable with respect to the bearing (5).

16. Measuring apparatus according to the claims 5 and 15 wherein the carrier (1.1, 1.2, 1.3) is tiltable with respect to the bearing (5) around the axis Y and/or the axis Z by means of the tilting actuator.

17. Measuring apparatus according to one of the preceding claims, wherein the measuring units (26) comprise micro projectors (8) and micro cameras (9), and particularly wherein the micro projectors (8) and micro cameras (9) are parts of the measuring head (1) and/or wherein the micro projectors (8) and micro cameras (9) are arranged alternatingly both line-by-line and column by column in Arrays, particularly wherein the micro projectors (8) generate a pattern of crossed stripes.

18. Measuring apparatus according to claims 5 and 17, wherein the micro projectors (8) and/or micro cameras (9) take different angular positions relative to the axis X, particularly in a range of 2 - 45°, and/or wherein piezo actuators are provided for deflecting the micro projectors and/or micro cameras individually or in groups.

19. Measuring apparatus according to one of the preceding claims, with at least one contact- or distance sensor (13.1, 13.2) to detect a contact of the measuring head (1) with the oral cavity to be measured, and particularly wherein the measuring apparatus is structured such that it varies a configuration of the carrier depending on the signal of the contact- or distance sensor (13.1, 13.2).

20. Measuring apparatus according to one of the preceding claims, for measuring at least a part of a row of teeth, wherein
the measuring head (1) defines a measuring space which is open on both sides along an axis X, is confined on both sides along an axis Y by the measuring head (1) and is open along an axis Z on one side and confined on one side by the measuring head (1), wherein the axes X, Y and Z are arranged orthogonal with respect to each other, and
wherein the measuring head (1) comprises:
- at least two lateral guiding members (2.1, 2.2) with guiding zones for the lateral abutting on the row of teeth, and
- at least two lateral measuring members (1.2, 1.3), wherein at least a part of the measuring units (26) is arranged in the lateral measuring members (1.2, 1.3),
and wherein the measuring head (1) is configured or configurable such that along the axis Z the distance (Dz) of the guiding zones from a part of the measuring member (1) confining the measuring space along axis Z is between 5 and 20 mm and that along the axis Y the distance (Dy) of the guiding zones of the lateral guiding members (22.2, 22.3) from each other is between 10 and 24 mm, particularly 14 and 22 mm.

21. Method for measuring at least a part of an oral cavity with an apparatus according to one of the preceding claims comprising the steps of
a) positioning of the measuring head (1) above a structure of the oral cavity, particularly above a row of teeth, such that the structure is placed in a measuring space (6) of the measuring head, and particularly wherein the measuring members (1.1, 1.2, 1.3) get in contact with the structure at least with their lateral guiding members (2.1, 2.2),
b) guiding of the measuring head along the structure, particularly such that at least one lateral guiding member stays in touch with the structure, and
c) measuring of the structure, particularly during the guiding of the measuring head.

22. Method according to claim 21, wherein the measuring apparatus comprises a shaft (4) and an electronically controlled tilting actuator (3) to tilt the measuring head (1) around at least one axis with respect to the shaft (4),
wherein during the measuring of the structure the measuring head is tilted with respect to the shaft (4) of the measuring apparatus into different positions by means of the tilting actuator, to generate measurements from different viewing angle positions.

23. Method according to one of the claims 21 or 22, wherein the configuration of the carrier is varied during the guiding of the measuring head (1) along the structure.

24. Method according to one of the claims 21 to 23, wherein the measuring head (1) as well as a shaft (4) of the measuring apparatus comprises occlusal guiding members (1.1.1; 5) for abutting on a row of teeth and wherein during the measuring of the row of teeth occlusal guiding members (1.1.1; 5) occlusally abut on the row of teeth.

25. Method according to claim 24, wherein the measuring head (1) comprises lateral guiding members (2.1, 2.2) and the lateral guiding members (2.1, 2.2) are brought into abutting against lateral surfaces of teeth of the row of teeth during the measuring or wherein the lateral guiding members (2.1, 2.2) are brought into abutting against the oral mucosa of the row of teeth during measuring.

26. Method according to one of the claims 21 to 25, wherein for measuring the measuring head (1) is guided without settling at least along half of a row of teeth, particularly along a complete row of teeth.

27. Method according to claim 26, wherein the measuring head (1) is guided in two runs from a left into a right region of the oral cavity and from the right into the left region of the oral cavity, or vice versa, wherein the measuring head (1) is taken off between the runs, is reversed by 180°, and put on again.

28. Method according to one of the claims 21 to 27, wherein the guiding of the measuring head (1) is carried out continuously, and particularly wherein the measuring apparatus (1) indicates via output means whether a guiding speed is correct,
or wherein the displacement of the measuring head (1) is carried out intermittently, particularly wherein the measuring apparatus indicates via output means, when the measuring head (1) is to be advanced further.

## Revendications

1. Appareil de mesure pour mesurer au moins une partie d'une cavité orale, particulièrement au moins une partie d'une rangée de dents, avec une tête de mesure (1), la tête de mesure (1) comprenant
au moins deux unités de mesure (26), chaque unité de mesure (26) ayant une zone de mesure et les zones de mesure étant arrangées de sorte qu'elles acquièrent des différentes sections de la partie d'une rangée de dents, et
un support (1.1, 1.2, 1.3; 19) qui porte les unités de mesure (26),
**caractérisé en ce que** chaque unité de mesure (26) est capable d'acquérir de manière trois-dimensionnelle au moins une surface dans son échelle de mesure, un arrangement relatif des échelles de mesure d'au moins deux des unités de mesure (26) étant déplaçable.

2. Appareil de mesure selon la revendication 1, le support étant apporté au moins dans une première et une deuxième configuration, l'agencement relatif des zones de mesure d'au moins deux des unités de mesure (26) étant différent dans la première et la deuxième configuration.

3. Appareil de mesure selon la revendication 2, le support (1.1, 1.2, 1.3; 19) étant au moins partiellement déformable de manière élastique ou plastique afin de le convertir de façon réversible de la première en la deuxième configuration et de la deuxième en la première configuration.

4. Appareil de mesure selon l'une des revendications 2 ou 3, le support (1.1, 1.2, 1.3; 19) ayant au moins deux membres de mesure (1.1, 1.2, 1.3) qui sont déplaçables l'un par rapport à l'autre, au moins une unité de mesure (26) étant arrangée à chaque membre de mesure (1.1, 1.2, 1.3).

5. Appareil de mesure selon l'une des revendications précédentes, la tête de mesure (1) définissant un espace de mesure (6) qui est ouvert des deux côtés le long de l'axe X, qui est limité des deux côtés par la tête de mesure (1), et qui est ouvert d'un côté et limité d'un côté par la tête de mesure (1) le long un axe Z dans au moins une configuration du support, les axes X, Y, Z étant arrangées de manière orthogonale l'un par rapport à l'autre.

6. Appareil de mesure selon la revendication 5, la tête de mesure (1) comprenant:
au moins deux membres de guidage latéraux (2.1, 2.2) avec des zones de guidage pour l'aboutement latéral sur la rangée de dents et
au moins deux membres de mesure latéraux (1.2, 1.3), au moins une partie des unités de mesure (26) étant arrangée dans les membres de mesure latéraux (1.2, 1.3),
et particulièrement la tête de mesure (1) étant configurée ou configurable de sorte que le long de l'axe Z la distance (Dz) des zones de guidage des membres de guidage (2.1, 2.2) d'une partie (1.1.1) du corps de guidage (1) qui limite l'espace de mesure le long de l'axe Z est comprise entre 5 et 20 mm et le long de l'axe Y la distance mutuelle (Dy) des zones de guidage des membres de guidage latéraux (2.1, 2.2) l'un par rapport à l'autre est comprise entre 10 et 24 mm, particulièrement 14 et 22 mm.

7. Appareil de mesure selon la revendication 6, les membres de mesure latéraux (1.2, 1.3) et/ou les membres de guidage latéraux (2.1, 2.2) étant arrangés de manière pivotable l'un par rapport à l'autre, et particulièrement les membres de mesure latéraux (1.2, 1.3) et/ou les membres de guidage latéraux (2.1, 2.2) étant arrangés de manière pivotable l'un par rapport à l'autre par au moins 30°, particulièrement au moins 90°.

8. Appareil de mesure selon la revendication 7, avec au moins un capteur de position (16.1, 16.2) à l'aide duquel une position de pivotement des membres de mesure latéraux (1.2, 1.3) et/ou des membres de guidage latéraux (22.2, 22.3) est mesurable.

9. Appareil de mesure selon l'une des revendications 6 à 8, la tête de mesure (1) comprenant en outre un membre de mesure central (1.1) auquel les membres de mesure latéraux (1.2, 1.3) sont arrangés de manière pivotable,
et particulièrement
- une distance des membres de guidage latéraux (2.1, 2.2) étant variable par au moins 3 mm et/ou
- les membres de mesure latéraux (1.2, 1.3) étant arrangés de manière transversale, particulièrement perpendiculaire, par rapport au membre de mesure central (1.1).

10. Appareil de mesure selon la revendication 9, un élément de support (1.1.1) étant arrangé d'un côté en face de l'espace de mesure (6) du membre de mesure central (1.1), qui est transparent ou encastré dans la zone des unités de mesure (26).

11. Appareil de mesure selon l'une des revendications 6 à 10, avec un entraînement (15.1, 15.2) pour pivoter les membres de mesure latéraux (1.2, 1.3) l'un par rapport à l'autre,
et particulièrement l'appareil de mesure comprenant des capteurs (13.1 - 13.14, 16.1, 16.2) pour commander l'entrainement (15.1, 15.2) et/ou les membres de mesure latéraux (1.2, 1.3) pouvant être pivotés dans une position prédéfinie à l'aide de l'entrainement (15.1, 15.2) .

12. Appareil de mesure selon l'une des revendications 6 à 11, les membres de mesure latéraux (1.2, 1.3) s'étendant le long de l'axe X sur une longueur de 3 à 12 mm, particulièrement 4 à 8 mm et/ou les membres de guidage latéraux (22.2, 22.3) s'étendant le long de l'axe X sur une longueur de 4 à 30 mm, particulièrement 6 à 16 mm.

13. Appareil de mesure selon l'une des revendications 6 à 12, les membres de guidage latéraux (2.1, 2.2) étant arrangés aux membres de mesure latéraux (1.2, 1.3), particulièrement le long des bords des membres de mesure latéraux (1.2, 1.3) tels que sont opposés au membre de mesure central (1.1).

14. Appareil de mesure selon l'une des revendications précédentes, une tige (4) étant arrangée à la tête de mesure (1), avec une longueur d'au moins 5 cm, particulièrement d'au moins 10 cm, et particulièrement une connexion mobile (12) étant arrangée entre la tige (4) et la tête de mesure (1), et particulièrement la tige (4) étant rotative autour de l'axe Z par rapport à la tête de mesure (1) par au moins 60°.

15. Appareil de mesure selon l'une des revendications précédentes, avec
un support (5) pour stabiliser la tête de mesure (1) sur une rangée de dents et
un entrainement inclinable (3) à l'aide duquel le support (1.1, 1.2, 1.3) est automatiquement inclinable par rapport à l'appui (5).

16. Appareil de mesure selon les revendications 5 et 15, le support (1.1, 1.2, 1.3) étant inclinable par rapport à l'appui (5) autour de l'axe Y et/ou Z à l'aide de l'entrainement inclinable.

17. Appareil de mesure selon l'une des revendications précédentes, les unités de mesure (26) comprenant des micro-projecteurs (8) et micro-caméras (9), et particulièrement les micro projecteurs (8) et les micro-caméras (9) faisant partie de la tête de mesure (1) et/ou les micro-projecteurs (8) et les micro-caméras (9) étant arrangés de manière alternante ligne-par-ligne et colonne-par-colonne dans des ensembles, particulièrement les micro-projecteurs (8) générant un motif de bandes croisées.

18. Appareil de mesure selon les revendications 5 et 17, les micro-projecteurs (8) et/ou les micro-caméras (9) prenant des différentes positions angulaires par rapport à l'axe X, particulièrement dans une gamme comprise entre 2 et 45°, et/ou des piézo-actionneurs étant prévus pour dévier les micro-projecteurs et/ou les micro-caméras individuellement ou en groupes.

19. Appareil de mesure selon l'une des revendications précédentes, avec au moins un capteur de contact ou de distance (13.1, 13.2) pour détecter un contact de la tête de mesure (1) avec la cavité orale à mesurer, et particulièrement l'appareil de mesure étant structuré de sorte qu'il varie une configuration du support dépendant du signal du capteur de contact ou de distance (13.1, 13.2).

20. Appareil de mesure selon l'une des revendications précédentes, pour mesurer au moins une partie d'une rangée de dents,
la tête de mesure (1) définissant un espace de mesure qui est ouvert des deux côtés le long d'un axe X qui est limité des deux côtés par la tête de mesure (1) le long d'un axe Y, et qui est ouvert d'un côté et limité d'un côté par la tête de mesure (1) le long d'un axe Z, les axes X, Y, Z étant arrangés de manière orthogonale l'un par rapport à l'autre, et
la tête de mesure (1) comprenant:
- au moins deux membres de guidage latéraux (2.1, 2.2) avec des zones de guidage pour l'aboutement latéral sur la rangée de dents, et
- au moins deux membres de mesure latéraux (1.2, 1.3), au moins une partie des unités de mesure (26) étant arrangée dans les membres de mesure latéraux (1.2, 1.3),
et la tête de mesure (1) étant configurée ou configurable de sorte que le long de l'axe Z la distance (Dz) des zones de guidage d'une partie (1.1.1) du corps de guidage (1) qui limite l'espace de mesure le long de l'axe Z est comprise entre 5 et 20 mm et le long de l'axe Y la distance (Dy) des zones de guidage des membres de guidage latéraux (22.2, 22.3) l'un par rapport à l'autre est comprise entre 10 et 24 mm, particulièrement 14 et 22 mm.

21. Procédé de mesure d'au moins une partie d'une cavité orale avec un appareil selon l'une des revendications précédentes comprenant les étapes
a) de positionner la tête de mesure (1) au-dessus d'une structure de la cavité orale, particulièrement au-dessus d'une rangée de dents, de sorte que la structure est placée dans un espace de mesure (6) de la tête de mesure, et particulièrement les membres de mesure (1.1, 1.2, 1.3) contactant la structure au moins avec leurs membres de guidage latéraux (2.1, 2.2),
b) de guider la tête de mesure le long de la structure, particulièrement de sorte qu'au moins un membre de guidage latéral reste en contact avec la structure, et
c) de mesurer la structure, particulièrement pendant le guidage de la tête de mesure.

22. Procédé selon la revendication 21, l'appareil de mesure comprenant une tige (4) et un entrainement inclinable (3) commandé électroniquement pour incliner la tête de mesure (1) autour d'au moins un axe par rapport à la tige (4),
pendant la mesure de la structure la tête de mesure étant inclinée par rapport à la tige (4) de l'appareil de mesure dans des positions différentes à l'aide de l'entrainement inclinable, afin de générer des mesures à partir des différentes positions d'angle de vue.

23. Procédé selon l'une des revendications 21 ou 22, la configuration du support étant variée pendant le guidage de la tête de mesure (1) le long de la structure.

24. Procédé selon l'une des revendications 21 à 23, la tête de mesure (1) ainsi qu'une tige (4) de l'appareil de mesure comprenant des membres de guidage occlusaux (1.1.1; 5) pour aboutir sur une rangée de dents et pendant la mesure de la rangée de dents des membres de guidage occlusaux (1.1.1; 5) aboutissant sur la ligne de dents de manière occlusale.

25. Procédé selon la revendication 24, la tête de mesure (1) comprenant des membres de guidage latéraux (2.1, 2.2) et les membres de guidage latéraux (2.1, 2.2) étant apportés en aboutissement contre des surfaces latérales des dents de la rangée de dents pendant la mesure ou les membres de guidage latéraux (2.1, 2.2) étant apportés en aboutissement contre la muqueuse orale de la rangée de dents pendant la mesure.

26. Procédé selon l'une des revendications 21 à 25, la tête de mesure (1) étant guidée pour la mesure au moins le long d'une moitié d'une rangée de dents sans arrêter, particulièrement le long d'une rangée de dents entière.

27. Procédé selon la revendication 26, la tête de mesure (1) étant guidée en deux passages à partir d'une région de gauche dans une région de droite de la cavité orale et à partir d'une région de droite dans une région de gauche de la cavité orale, ou vice versa, la tête de mesure (1) étant enlevée entre les passages, inversée par 180°, et introduite de nouveau.

28. Procédé selon l'une des revendications 21 à 27, le guidage de la tête de mesure (1) étant effectué de manière continue, et particulièrement l'appareil de mesure (1) indiquant par des moyens de sortie si une vitesse de guidage est correcte,
ou le déplacement de la tête de mesure (1) étant effectué de manière intermittente, particulièrement l'appareil de mesure indiquant par des moyens de sortie quand la tête de mesure (1) doit être avancée.
